# EUROPEAN PATENT APPLICATION

(11) **EP 2 145 615 A1**
(43) Date of publication of application: **20.01.2010**
(21) Application number: 08739770.9
(22) Date of filing: 03.04.2008
(51) Int. Cl.: A61K 8/49, A23K 1/16, A23K 1/18, A23L 1/30, A61K 8/60, A61K 31/353, A61K 31/7048, A61P 1/02, A61P 3/04, A61P 3/06, A61P 3/10, A61P 5/30, A61P 7/00, A61P 9/00, A61P 11/00, A61P 15/12, A61P 17/00, A61P 17/10, A61P 17/14, A61P 17/16

(54) **SKIN-LIGHTENING AGENT CONTAINING EQUOL COMPOUND AS ACTIVE INGREDIENT**

(30) Priority: 09.04.2007 JP 2007102164
(71) Applicant: Kabushiki Kaisha Hayashibara Seibutsu Kagaku Kenkyujo, Kita-ku Okayama-shi Okayama 700-0907 (JP)
(72) Inventor: NAKANO, Masayuki, Kita-ku, Okayama-shi Okayama 700-0907 (JP); SHIBUYA, Takashi, Kita-ku, Okayama-shi Okayama 700-0907 (JP); MIYAKE, Masaki, Kita-ku, Okayama-shi Okayama 700-0907 (JP); ARAI, Norie, Kita-ku Okayama-shi Okayama 700-0907 (JP)
(74) Representative: Daniels, Jeffrey Nicholas
(86) International application number: PCT/JP2008/056663
(87) International publication number: WO 2008/126752

(57) **Abstract**

An object of the present invention is to provide a whitening agent containing a novel whitening substance as an effective ingredient. The above object is attained by providing a whitening agent containing equol and/or equol glycoside, in which one or more glycosyl groups bind to the hydroxyl group(s) of equol, as an effective ingredient.

## Description

### TECHNICAL FIELD

The present invention relates to a whitening agent comprising equol and/or equol compound as an effective ingredient(s).

### BACKGROUND ART

In an aging society, awareness of beautification and health has been increased. Particularly for women, flecks and dullness of the skin become problematic, and different types of cosmetics or quasi-drugs incorporated with various whitening substances have been commercialized. However, each one of these whitening substances may not exert enough effect when used alone, and an external dermatological agent prepared by incorporating two or more whitening substances in combination have been proposed (see Japanese Patent Kokai No. 284664/2002, for example). However, development of a novel whitening substance having safe and enough effect of whitening is expected.

Equol is a compound metabolically formed from daizein, one of isoflavones, by intestinal bacteria. Since it was found that estrogenic activity of isoflavone is enhanced by being converted into equol, it is proposed that equol is used for preventing and improving menopausal symptoms and osteoporosis (see Japanese Patent Kohyo No. 523258/2001), for improving and preventing aging and wrinkle of the skin (see Japanese Patent Kohyo No. 511860/2002), or for palliating allergic symptoms (see International Patent Publication No. WO 03/097037).

Two stereoisomers, S-isomer and R-isomer, of equol are known (hereinafter, S-isomer and R-isomer are described as "S-equol" and "R-equol", respectively). Equol can be produced by synthetic method or fermentative method (see Japanese Patent Kohyo No. 504409/2006, for example), and synthetically prepared equol is commercialized as a regent. Such synthetically prepared equol is a mixture of the above two stereoisomers. On the other hand, equol converted from daizein in human intestine consists of S-equol. Both of the two isomers (S-equol and R-equol) have estrogenic activity by binding to estrogen-receptor, however, it is considered that the estrogenic activity of S-equol is higher than that of R-equol according to the affinity to the receptor (Kenneth D. R. et al., American Journal of Clinical Nutrition, Vol. 81, pp. 1072-1079 (2005)). External dermatological compositions and foods and beverages having the above effect of equol have been already suggested (see Japanese Patent Kohyo No. 511860/2002 and Japanese Patent Kohyo No. 504409/2006), however, substantially no researches on other effects and applications of equol are found.

### DISCLOSURE OF INVENTION

An object of the present invention is to provide a whitening agent comprising a novel whitening substance as an effective ingredient.

The inventors of the present invention have energetically studied to attain the above object and unexpectedly found that equol has whitening activity by inhibiting melanin production in melanocyte. They also found that equol has enhancing effect on inducing differentiation of precursor lipocytes into lipocytes, and enhancing effect on lipid degradation in lipocytes. They also found that equol glycosides, in which one or more glycosyl groups bind to equol via α-linkage, exert the activity inherent to equol, such as the activities described above, estrogenic activity, antioxidant activity, and antiallergic activity; and the inventors accomplished the present invention.

The present invention attains the above object by providing whitening agent containing equol and/or equol glycoside, in which one or two or more glycosyl groups bind to equol via α-linkage, (hereinafter, equol and its glycoside maybe collectivelydescribedas "equol compound") . Equol compound as referred to as in the present invention includes any mixture of two or more kinds of equol and equol glycosides, in which equol itself or the equol moiety of the glycosides consists of either R-equol or S-equol, or comprises a mixture of both isomers. In terms of physiological activity, it is desirable that the content of S-equol and its gycosides in the total amount of equol compounds is higher than that of R-equol compounds. Hereinafter, S-equol and R-equol are collectively described as "equol".

Since equol compound as an effective ingredient exerts whitening effect, enhancing effect on induction of differentiation of precursor lipocytes into lipocytes, enhancing effect on lipid degradation in lipocytes (hereinafter, described as "lipid metabolism improving effect"), the equol compounds, or foods and beverages, medicines, quasi-drugs, cosmetics, and groceries comprising the equol compounds are usable for whitening and/or improving lipid metabolism.

The term "Whitening" as referred to as in the present invention includes preventative effect such as inhibiting tanning of the skin as well as active effect such as whitening the skin. Concretely, it includes effect of beautifying the skin such as keeping the skin and oral tissues including the gum beautiful by inhibiting pigmentation, as well as effect of palliating pigmentation on the skin such as flecks and freckles. The term "Lipid metabolism improving effect" includes enhancing effect on inducing differentiation of precursor lipocytes into lipocytes, and effect of reducing or regulating body fat such as visceral and subcutaneous fat or blood lipid such as triglyceride and cholesterol by enhancing lipid degradation in lipocytes.

### BRIEF EXPLANATION OF DRAWINGS

FIG. 1 shows an elution profile on analytical HPLC (using column ODS-AM-303) of the reaction product (equol glycosides), prepared by allowing glycosyltransferase to act on a mixture of equol and dextrin and treating the reaction mixture with glucoamylase, according to the method of the present invention.
FIG. 2 shows an elution profile on preparative HPLC (using column D-ODS-5 S-5) of the reaction product containing equol glycoside, prepared by purifying a reaction mixture, obtained by allowing glycosyltransferase to act on a mixture of equol and dextrin according to the method of the present invention, with a synthetic porous adsorption resin.
FIG. 3 shows an elution profiles on the preparative HPLC of the isolated products, obtained by subjecting the reaction product 'a' (4'-O-α-D-glucopyranosyl-S-equol) or the reaction product 'b' (7-O-α-D-glucopyranosyl-S-equol), prepared according to the method of the present invention, to the preparative HPLC.
FIG. 4 shows a mass spectrum of the reaction product 'a' (4'-O-α-D-glucopyranosyl-S-equol) prepared according to the method of the present invention, after eluted from preparative HPLC (using column D-ODS-5 S-5).
FIG. 5 shows a ¹H-NMR spectrum of S-equol.
FIG. 6 shows a ¹³C-NMR spectrum of S-equol.
FIG. 7 shows a ¹H-NMR spectrum of the reaction product 'a' (4'-O-α-D-glucopyranosyl-S-equol) prepared according to the method of the present invention.
FIG. 8 shows a ¹³C-NMR spectrum of the reaction product 'a' (4'-O-α-D-glucopyranosyl-S-equol) prepared according to the method of the present invention.
FIG. 9 shows a H-H COSY of the reaction product 'a' (4'-O-α-D-glucopyranosyl-S-equol) prepared according to the method of the present invention.
FIG. 10 shows a C-H COSY spectrum of the reaction product 'a' (4'-O-α-D-glucopyranosyl-S-equol) prepared according to the method of the present invention.
FIG. 11 shows a ¹H-NMR spectrum of the reaction product 'b' (7-O-α-D-glucopyranosyl-S-equol) prepared according to the method of the present invention.
FIG. 12 shows a ¹³C-NMR spectrum of the reaction product 'b' (7-O-α-D-glucopyranosyl-S-equol) prepared according to the method of the present invention.
FIG. 13 shows a H-H COSY spectrum of the reaction product 'b' (7-O-α-D-glucopyranosyl-S-equol) prepared according to the method of the present invention.
FIG. 14 shows a C-H COSY spectrum of the reaction product 'b' (7-O-α-D-glucopyranosyl-S-equol) prepared according to the method of the present invention.
FIG. 15 shows an LC/MS profile in the range of m/z 564 to 566, using ODS-AQ-303 column, of equol glycoside, prepared according to the method of the present invention, obtained by chromatography using a synthetic porous adsorption resin (HP-20).
FIG. 16 shows mass spectra of three peaks, whose retention times are 6.63 to 6.71 minutes (a), 6.77 to 6.83 minutes (b), and 7.09 to 7.21 minutes (c), on HPLC using ODS-AQ-303 column.

### BEST MODE FOR CARRYING OUT THE INVENTION

"Equol" as referred to as in the present invention means a compound having the structure represented by General Formula 1, in which both R₁ and R₂ are hydrogen atoms. "Equol glycoside" means a compound having the structure described in General Formula 1, in which either R₁ or R₂ is glycosyl group, both R₁ and R₂ are glycosyl groups, or mixture of them. The total number of monosaccharide consisting of glycosyl group of R₁ and R₂ is desirably one to six, preferably, one to four. When used as an external dermatological agent, oral medicine or quasi-drug, the number is desirably one in terms of specification setting. 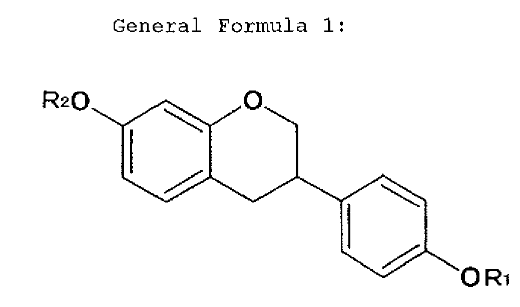 (R₁ and R₂ independently represent hydrogen atoms or glycosyl groups.)

The source or preparation method of the equol compound used in the present invention is not restricted. They can be prepared by an organic synthesis method, fermentation method, or combination thereof.

Any equol prepared by chemical synthesis method as disclosed in Japanese Patent *Kohyo* No. 504409/2006 including, for example, those which are prepared by fermentation method, or commercially-manufactured is usable in the present invention. Any one of the two stereoisomers, or a mixture thereof is usable.

As a process for producing the equol glycoside used in the present invention, enzymatic method, in which a glycosyltransferase is allowed to act on a mixture containing equol and α-glycosyl saccharide, is economically efficient and industrially preferable because equol glycoside can be produced in large quantity at low cost when equol and an inexpensive substrate such as starch or its partial hydrolysate are used. A process for producing equol glycoside using glycosyltransferase is explained below.

Any one of the equol described above is feasible as a material for preparing equol glycoside. When either of the equol glycosides is prepared from a mixture of S-equol and R-equol, S-equol or R-equol can be used after separated from the mixture, or the equol glycosides can be separated after S-equol and R-equol are glycosylated.

The glycosyltransferase used for preparing equol glycoside in the present invention is not restricted as long as it produces equol glycoside when allowedto act on a solution containing equol and α-glycosyl saccharide. Concretely, α-glucosidase, cyclomaltodetrin glucanotransferase (hereinafter, it may be abbreviated as "CGTase") and α-amylase can be used. The source and type of these enzymes are not restricted as long as they can produce equol glycoside used in the present invention, any enzyme prepared from animals, plants, fungi, bacteria or yeasts is usable, particularly, the enzyme from microorganism is preferable in terms of ease and safety of production, and CGTase is more preferable in terms of glycoside productivity. These enzymes should not necessary be purified ones. When the enzyme from a microorganism is used, for example, a crude enzyme such as a culture of microorganisms or its concentrate or salted-out precipitate is feasible for attaining the present invention. If necessary, any enzyme purified by conventional method or any enzyme immobilized on a carrier is usable. Also a commercially available glycosyltransferase can be used. Usually, the amount of enzyme is defined to complete the reaction within about five to 80 hours in terms of economy. When an immobilized enzyme is used, either batch or continuous method is arbitrarily adopted.

As α-glycosyl saccharide for preparing equol glycoside used in the present invention, any compound usable as a substrate of glycosyltransferase in preparation of equol glycoside is used, for example, partial starch hydrolysates such as amylose, dextrin, cyclodextrin and maltooligosaccharide, gelatinized starch, and liquefied starch can be used. To facilitate the preparation of equol glycoside, suitable α-glycosyl saccharide for the glycosyltransferase can be selected depending on the enzyme used.

The concentration of α-glycosyl saccharide in a solution for transglycosylation is not restricted, it is usually 0.1% (w/w) (hereinafter, % (w/w) is described as "%" in this specification, unless specified otherwise) or higher, preferably, 0.1% to 70%, more preferably, 0.1% to 50%. Equol solution for the reaction desirably contains equol at a higher concentration as much as possible. For example, solutions containing equol at a higher concentration, in which equol is suspended or dissolved at a higher temperature, or dissolved at an alkaline pH are feasible, and the equol concentration is desirably about 0.005% or higher, more preferably, about 0.01% to 10.0%.

In the reaction method of the present invention, it is desirable that glycosyltransferase is allowed to act on equol at a higher concentration. When suspended equol is used, for example, a solution containing equol in suspension at a concentration of 0.1% to 2.0% and an adequate amount of α-glycosyl saccharide is adjusted to about pH 4.0 to 7.0, kept at a temperature as high as the glycosyltransferase can work, concretely about 20°C to 90°C, and the glycosyltransferase is allowed to act on, the suspended equol become solubilized by being converted to equol glycoside and equol glycoside is easily obtained at a higher concentration.

Optionally, to make the transglycosylation reaction to equol easy by elevating the concentration of equol before the reaction, a solution containing equol at a higher concentration can be admixed with an organic solvent mutually soluble in water, such as lower alcohols or lower ketones including methanol, ethanol, n-propanol, isopropanol, n-butanol, acetonitrile and acetone.

Although a relatively high molecular equol glycoside formed after the transglycosylation is usable without modifications, if necessary, it can be used after purified with a synthetic porous absorptive resin, equol glycoside with reduced number of glucose in the α-glycosyl group can be obtained by hydrolysis with amylase such as glucoamylase (EC 3.2.1.3) and β-amylase (EC 3.2.1.2). For example, when glucoamylase is used, α-glucosyl equol is produced along with glucose by hydrolysis of higher molecular glycosides than α-maltosyl equol. When β-amylase is used, a mixture of α-glucosyl equol and α-maltosyl equol is produced along with maltose by hydrolyzing higher molecular glycosides than α-maltotriosyl equol.

By the process described above, the following six kinds of equol glycosides can be obtained: 4'-O-α-D-glycosyl-equol, in which glycosyl group binds to the OH group at C-4' of S-equol or R-equol, 7-O-α-D-glycosyl-equol, in which glycosyl group binds to the OH group at C-7 of S-equol or R-equol, 4',7-O-α-di-D-glycosyl-equol, in which glycosyl groups bind to both the OH groups at C-4' and C-7 of S-equol or R-equol. The number of glucose molecule in the glycosyl group is not restricted, it is usually desirably one to six, preferably, one to four, more preferably one, in terms of productivity and usability. The reaction mixture obtained by the process of the present invention, for example, containing glycoside made from S-equol and R-equol, is usable in any form such as the preparation without modification, partially or highly purified preparation, or three types of glycosides withdifferentbinding positions of glycosyl groups isolated by fractionation, mixtures of isolated fractions, syrupy preparations obtained by concentration of the mixtures, driedproducts, and pulverized products, as a whitening agent and/or lipid metabolism improving agent, material of external dermatological composition, or orally-administered composition.

When the solution containing equol glycoside is pulverized, the drying method and pulverizing method are not restricted. The solution is dried without modification or after admixed with a base of pulverization such as saccharides including dextrin, by freeze-drying, spray-drying, or drum drying method. Among them, freeze-drying and spray-drying method, particularly spray-drying method is desirable because the powdery product can be easily produced in large quantity at a lower cost. For the spray drying, rotating diskmethod, pressured nozzle method, or two-fluid nozzle method can be arbitrarily adopted.

The purification method of the equol glycosides is not restricted as long as the purity of equol glycoside is elevated. Usually, equol glycoside is purified by separation from other substances such as α-glycosyl saccharide according to difference of adsorbability to synthetic porous absorptive resin. When equol glycoside without removal of unglycosylated equol is used, the duration of the activity of equol compounds can be prolonged by time-lag in metabolism of the equol and equol glycoside, because equol itself has whitening effect and lipid metabolism improving effect. In terms of water solubility, the content of equol glycoside is desirably elevated as high as possible.

Examples of the synthetic porous absorptive resins used for purifying the equol glycoside include the following nonionic synthetic resins having a large adsorption area and high porousness, such as styrene-divinylbenzen polymers, phenol-formaldehyde resins, acrylate resin and m-acrylate resins: "AMBERLITE XAD-1", "AMBERLITE XAD-2", "AMBERLITE XAD-4", "AMBERLITE XAD-7", "AMBERLITE XAD-8", "AMBERLITE XAD-11", and "AMBERLITE XAD-21", produced by Rohm & Haas Company; "DIAION HP-10", "DIAIONHP-20", "DIAIONHP-30", "DIAIONHP-40", and "DIAION HP-50", produced by Mitsubishi Chemical Industries Limited; and "IMACTI Syn-42", "IMACTI Syn-44", and "IMACTI Syn-46", produced by IMACTI Company.

When the reaction solution containing the equol glycoside is applied to a column packed with the above synthetic porous absorption resin, equol glycosides and residual equol, remained in the applied solution in a relatively small amount, adsorb on the resin, while most of the α-glycosyl saccharides, contained in the applied solution in a large amount, elute without adsorbtion.

If necessary, after the reaction with glycosyltreanferase but before applying to the synthetic porous absorptive resin, the solution can be arbitrarily treated with purification method such as filtering out insoluble substances formed in heating treatment of the reaction solution, removing proteinaceous substances in the solution by adsorbing the solution on magnesium aluminosilicate or aluminosilicate, desalting the solution by treatment with a strongly acidic cation exchange resin (H-form), anion exchange resin or weakly basic anion exchange resin (OH-form), or their combination.

After the equol glycosides and a relatively small amount of unreacted equol, adsorbing on the synthetic porous absorption resin, were washed with dilute alkaline solvents or water, firstly equol glycosides are eluted with a relatively small amount of orgaic solvent or a mixture of water such as aqueous methanol or aqueous ethanol, secondly unreacted equol is eluted by elevating the amount of eluent or elevating the concentration of the organic solvent.

Since the elution process of equol glycosides and unreacted eauol doubles as the regeneration process of the synthetic porous absorption resins, it enables repeating use of the resins.

After the organic solvent is removed from the eluent containing equol glycosides at a higher concentration by distillation, a syrupy product containing equol glycosides as main components is obtained by concentrating up to an adequate concentration. Furthermore, a powdery product containing equol glycosides as main components is obtained by drying and pulverizing the syrupy product by freeze drying method or spray drying method.

Also other water soluble substances such as salts can be removed as well as α-glycosyl saccharide by purification with synthetic porous adsorption resins in the present invention. Since any of 4'-O-α-D-glycosyl equol, 7-O-α-D-glycosyl equol and/or 4',7-O-α-di-D-glycosyl equol has a relatively high water-solubility, they are usable in any form such as a mixture thereof without modification, isolated preparation of either of the three equol glycosides, or a mixture of the isolated preparations, as the whitening agent and/or the lipid metabolism improving agnent of the present invention, or whitening composition and/or the lipid metabolism improving composition containing the agent.

The equol compounds and the whitening agent and/or the lipid metabolism improving agent containing the compounds are added to a composition in its manufacturing process before completion or to the final product, concretely by a method such as mixing, kneading, dissolving, melting, dispersing, suspending, emulsifying, permeating, crystallizing, interspersing, applying, adhering, spraying, coating, injecting, soaking, solidifying and arbitrary combination of one or more of the above methods.

The amount of equol compounds in the composition of the present invention is not restricted as long as it exerts physiological effect of the equol compounds such as whitening effect or lipid metabolism improving effect, usually, the content in equol equivalents is desirably 0.0001% to 10 %, preferably, 0.001% to 10%, more preferably, 0.05 to 5%, much more preferably, 0.1% to 2%. Within the above range, the physiological effect of equol is exerted. When it is lower than 0.0001%, the physiological effect of equol is not exerted. When it is higher than 10%, physical problems such as discoloration or deterioration of usability may occur according to the composition. "Equol Equivalent" as referred to as in the present invention means that the amount of equol glycoside is represented as the amount of equimolar equol.

The whitening agent and/or the lipid metabolism improving agent containing equol compound of the present invention and the composition containing the agent are usable in a product form such as foods and beverages, medicines, quasi-drugs, cosmetics, feedstuff, baits or pet foods. Particularly, the desirable form is an external dermatological composition such as medicines, quasi-drugs or cosmetics; or orally-administeable composition such as foods and beverages, medicines or quasi-drugs. Since the equol compound also has estrogenic activity, antioxidant activity and antiallergic activity as well as the effects described above, the compositions are arbitrarily used for antiaging, preventing and treating osteoporosis or menopausal disorder in addition to preventing, palliating or treating life-style disease or metabolic syndrome. Since equol compound has lipid metabolism improving effect, it can be advantageously used as a preventive agent of swelling because it also exerts slimming effect by reducing local body fat when used as an external dermatological agent.

"External dermatological composition" as referred to as in the present invention means an external agent applied to the exodermis such as the skin and the scalp, and the oral cavity, in an agent form of, for example, ointment, cream, face lotion, milky lotion, lotion, essence, jelly, gel, pack, mask, bath agent, cleaning agent, makeup cosmetics, hair-care cosmetics, dispersion liquid, liquid agent, aerosol agent, adhesive agent, adhesive skin patch, liniment, powdery dentrifice, wet denttifice, paste dentrifice, loquid dentrifice, medical dentrifice, mouth refrigerant, mouth refrigerating film, mouth wash and gargle.

The external dermatological composition is admixed with following materials available for compositions such as foods and beverages, cosmetics, quasi-drugs and medicines, as long as the effect of the present invention is not inhibited: waters (including purified water, spring water, deep sea water), alcohols, oils, surfactants, metal soaps, gelling agents, powders, water-soluble polymers, saccharides, membrane-forming agents, resins, ultraviolet (hereinafter, abbreviated as "UV") protective agents, clathrate compounds, antimicrobial agents, antiseptics, flavors, dyes, deodorants, salts, pH regulators, algefacients, extract of animal or microbial, plant extracts, blood circulation improving agents, anti-inflammatory agents, astringent agents, antiseborrheic agents, whitening agents, active oxygen eliminating agents, cell activating agents, humectants, chelating agents, keratolytic agents, enzymes, hormones, vitamins and minerals. Concretely, following materials are quoted.

The alcohols are used for dissolving, giving coolness, preservation or moisturizing. Such alcohols include lower alcohols such as ethanol, polyalcohols such as glycerol, diglycerol, polyglycerol, ethyleneglycol, diethyleneglycol, prolyleneglycol, diprolyleneglycol, 1,3-butyleneglycol, pentyleneglycol, polyethyleneglycol and isopentyldiol; and sugar alcohols such as sorbitol, maltitol, maltotriitol, maltotetraitol and maltopentaitol.

The oils are used as components of base materials to improve usability or comfort. Any oil available for cosmetics is usable independently of its origin such as natural oil or synthetic oil, or its form such as solid, semisolid or liquid. Examples of such oils include hydrocarbons, waxes, fatty acids, higher alcohols, esters, silicone oils and fluorinated oils. More concretely, the following oils are quoted: hydrocarbons such as liquid paraffin, squalene and vaseline, synthetic esters such as cetyl tri-2-etylhexanate, and pentaerythryl tetra-2-ethylhexanate; fats and oils from plants or animals such as olive oil, castor oil, jojoba oil, mink oil, macadamia nut oil, amygdule oil, persic oil, safflower oil, sunflower oil, avocado oil, meadfoam oil, camellia oil, almond oil, perilla oil, sesame oil, borage oil, and shia oil; and waxes such as beeswax, carnauba wax, cancerilla wax and whale wax.

The following materials are usable as UV-protective agents: *p*-aminobenzoic acid and its esters, octyl salicylate, homomenthyl salicylate, methyl 2,5-diisopropylcinnamate, cinoxate, glycerol di-*p*-methoxycinnamate mono-2-ethylhexanoate, dihydroxymethoxybenzophenone, sodium dihydroxymethoxybenzophenone disulfonate, dihydroxybenzophenone, 1-(3,4-dimethoxyphenyl)-4,4-dimethyl-1,3-pentadione, dimethicodiethylbenzomalonate and its esters, 2-ethylhexyl dimethoxybenzylidenedioxoimidazolidinepropionate, tetrahydroxybenzophenone, 2,4,6-tris[-(2-ethylhexyloxycarbonyl)anilino)-1,3,5-triazine, methyl-bis-(trimethylsiloxy)-silylisopentyl trimethoxycinnamate, amyl p-dimethylaminobenzoate, 2-ethylhexyl p-dimethylaminobenzoate, dietylaminohydroxybenzohexylbenzoic esters, mixture of p-methoxycinnamic esters, 2-etylhexyl p-methylcinnamate, 2-hydroxy-4-methoxybenzophenone, hydroxymethoxybenzophenonesulfonic acid and its tetrahydrate, sodium hydroxymethoxybenzophenonesulfonate, phenylbenzimidazolesulfonic acid, 4-tert-butyl-4-methoxybenzoylmethane, 2-ethylhexyl 2-cyano-3,3-diphenylpropan-2-enoate (octocrylene), 4-(2-β-glucopyranoxyloxy)propoxy-2-hydroxybenzophenone, terephthalylidene dicamphor sulfonic acid, ferulic acid, 2,2'-methylene-bis-(6-(2H-benzotriazole-2-yl)-4-(1,1,3,3-tetrametylb utyl)-phenol, metrizoltrisiloxane, titanium oxide and zinc oxide.

Water-soluble polymers and saccharides are used for stabilizing the composition of the present invention, improving the usability or comfort, or attaining moisturizing effect. The following water-soluble polymers are usable: Pullulan, plant polymers such as carrageenan, pectin, agar and locust bean gum, cellulosic polymers such as methylcellulose, carboxymethylcellulose and hydroxypropylcellulose, alginic polymers such as sodium alginate, and vinylpolymers such as carboxyvinylpolymers. The following saccharides are usable : Maltose, maltotriose, maltotetraose, trehalose, saccharide derivatives of trehalose and cyclic tetrasaccharides.

The following compounds are quoted as antiseptics and antimicrobial agents: benzoic acid, sodium benzoate, p-oxybenzoic ester, benzalkonium chloride, phenoxyehtanol, isopropylmethylphenol and pentandiol.

Although the equol compound exerts beneficial effect of whitening and improving lipid metabolism, to improve the effects much more, the whitening agents of the present invention and the compositions containing them can be admixed with known whitening agents or lipid metabolism improving agents. The known whitening agents are used for preventing tanning caused by sunburn or fleck or freckle caused by pigmentation, or lowering or inhibiting local fat accumulation. The following known whitening agents are usable: Arbutin, ellagic acid, linoleic acid, 5,5'-dipropyl-bis-phenyl-2,2'-diol, alkylresorcinol, vitamin C, vitamin E, vitamin P, glycyrrhizic acid, tranexamic acid, koji acid and their derivatives, tetrahydrocurcuminoid, placenta extract, indigo plant extract, camomilla extract, licorice extract, rose fruit extract, Scutellaria extract, seaseed extract, *Sophorae radix* extract, leguminosa sarmentum extract, acanthopanasic bark extract, rice bran extract, wheat germ extract, saiasarum root extract, hawthorn extract, *Cassia mimosoides* extract, white lily extract, peony extract, *Inula britannica* extract, soybean extract, tea extract, molasses extract, *Ampelopsis japonica* extract, grape extract, hop extract, *Rosa rugosa* extract, *Chaenomeles speciosa* extract, saxifrage extract, coix seed extract, and royal jelly extract.

The anti-inflammatory agents are used for relieving inflammation such as skin-burning and erythema after sunburn. The following compounds are usable as the anti-inflammatory agents: Sulfur and its derivatives, glycyrrhizic acid and its derivatives, glycyrrhetic acid and its derivatives, levocarnitine chloride, aloe extract, althea extract, angelica extract, arnica extract, indigo plant extract, *Artemisia capillaris* extract, nettle extract, phellodendron bark extract, *Hypericum erectum* extract, camomilla extract, licorice extract, *Lonicera japonica* extract, cress extract, comfrey extract, sage extract, lithospermum root extract, perilla extract, white birch extract, and gentian extract.

The cell activating agents are used for relieving rough dry skin. The following compounds are usable as the cell activating agents: Caffeine, cock's comb extract, shell extract, shellfish extract, royal jelly, silk protein and its decomposed product and their derivatives, lactoferrin and its decomposed product, mucopolysaccharide such as chondroitin sulfuric and hyaluronic acid and their salts, collagen, yeast extract, lactic bacteria extract, bifidobacteria extract, fermentation-metabolic product, ginkgo extract, barley extract, sialid extract, jujube extract, gensing extract, rosemary extract and organic acids such as glycolic acid, citric acid, lactic acid, malic acid, tartaric acid, and succinic acid including their derivatives.

The active oxygen eliminating agents are used for inhibiting oxidative affection such as production of lipid peroxide. The following compounds are usable as the active oxygen eliminating agents: Superoxide dismutase, mannnitol, quercetin, catechin and its derivatives, rutin and its derivatives, moutan bark extract, cornflower seed extract, melissa extract, *Shiraitia grosvenorii* extract, vitamins A such as retinal, carotenoids and their derivatives, vitamins B such as thiamin, riboflavin, pyridoxine, nicotinic acid and their derivatives, vitamin C and its derivatives, vitamins P such as rutin, hesperidin, naringin and their derivatives, vitamins E such as tocopherol and its derivatives, dibutylhydroxytoluene, and butylhydroxyanisole.

The following compounds are usable as the humectants: Proteins such as elastin, keratin and their derivatives, hydrolysate and salts, amino acids such as glycine, serine, asparatic acid, glutamic acid, arginine, theanine and their derivatives, saccharine such as sorbitol, erythritol, trehalose and its derivatives, inositol, glucose, maltose, sucrose and its derivatives, dextrin, cyclodextrin, cyclic tetrasaccharide and its derivatives and honey, D-panthenol and its derivatives, urea, phospholipids, ceramide, *Coptis rhizome* extract, calamus extract, rehmannia root extract, *Cnidium rhizome* extract, tree mallow extract, thyme extract, cordata extract, hamamelis extract, lime tree extract, horse chestnut extract and quince extract.

The equol compound, which is orally administered alone as the whitening agent and/or lipid metabolism improving agent, also advantageously used as a material for preparation of whitening and/or lipid metabolism improving compositions such as foods and beverages, quasi-drugs, medicines, feedstuffs, baits, pet foods, being admixed with one or more substances such as reducing saccharide, non-reducing saccharide, sugar alcohols, high-intensity sweeteners, water-soluble polysaccharides, organic acids, inorganic acids, salts, emulsifiers, antioxidant agents, chelating agents, known dyes, flavors, preservatives, acidifiers, *"umami"* seasonings, sweeteners, stabilizers, fillers, alcohols, and water-soluble polymers. The orally-administerable composition containing equol compound exerts whitening effect and lipid metabolism improving effect by oral administration. When ingested, the orally-administerable composition also exerts estrogenic physiological effect, antiaging, anti-osteoporosis, anti menopaudal disorder and preventive or improving effect on metabolic syndrome, diabetes, obesity, and heart-circulatory system disease.

The form of the orally-administerable composition of the present invention is not restricted, and the following form of one or more seasoning or sweetener are quoted: Amino acids, peptides, soy sauce, powdery soy sauce, *"miso"* (fermented soy paste), powdery *"miso", "moromi"* (unrefined soy), *"hishio", "furikake"* (flakes to sprinkle on cooked rice), mayonnaise, dressing, powdery vinegar for *"sushi"* (vinegared rice), Chinese seasoning, sauce, ketchup, grilled beef sauce, curry roux, instant stew, instant soup, instant bouillon, nucleic-acid based seasonings, mixed seasoning, *"mirin"* (sweet cooking rice wine), *"shin-mirin"* (syrupy sweet seasoning), table sugar and coffee sugar.

Also the following forms of various kinds of foods and beverages are quoted: rice confectioneries such as *"senbei", "arare"* and *"okoshi";* Japanese confectioneries such as *"kyuhi" "monaka",* rice cake, *"ohagi", "manju", "karukan", "uiro",* bean jam, *"yokan", "mizu-yokan", "kingyoku", "kintsuba",* sweet potato, jelly, bavarois, pao de Castella and drop; baked goods such as biscuit, cookie, cracker, pie, cream puff, waffle, sponge cake, donut and pastry; western confectioneries such as pudding, butter cream, custard cream, chocolate, chewing gum, soft candies including nougat, jellybean, gummy candy, caramel and marshmallow, hard candy, fondant and icing; snacks; cereal; center-liquid candy; meringue confectionery; breads such as sandwich loaf, baked roll, red bean bread and muffin; fruit preserved in syrup; syrups such as shaved ice syrup; pastes such as flour paste, peanut paste and fruit paste; processed fruits such as jam, marmalade, preserve, fruit soaked in syrup, candied fruit and cut fruit; sprouted vegetables such as bean sprout, alfalfa and sprouted broccoli; vegetable juices such as green juice; processed vegetables such as cut vegetable, salad and cooked vegetables; pickles and seasonings for preparing pickles; rice diets such as cooked polished rice, rice ball, rice with red beans, rice gruel, rice seasoned with vinegar, seasoned steamed rice with vegetables and meat and pregelatinized rice; processed beans such as soy milk, bean curd, freeze-dried bean curd and fermented soybean; noodles such as Japanese wheat noodle, buckwheat noodle, Chinese noodle and pasta; processed meat foods such as *"okonomiyaki"* (Japanese savory pancake), *"takoyaki"* (octopus dumpling), *"taiyaki"* (fish-shaped pancake stuffed with bean jam), croquette, "gyoza" (potsticker), *"shuumai"* (Chinese-style steamed meat or shrimp dumpling), *"harumaki"* (Chinese pancake roll), ham and sausage; processed fishery foods such as fish ham, fish sausage, *"kamaboko"* (a kind of steamed fish paste) and *"chikuwa"* (a kind of steamed fish paste) and deep fried seafood; delicacies such as sea urchin, salted fermented cuttlefish, vinegared sea tangle, splitting dried squid, *"mirin-boshi"* of blowfish, salmon caviar and seasoned dried seaweed; sauces for grilled beef, split and broiled eel, rice dumpling or rice cracker; *"tsukudani"* (boiled-in-soy sauce foods) of seaweed, wild vegetable, squid, small fry or shellfish; prepared foods such as cooked bean, potato salad and tangle roll; processed egg such as egg, omelet, rolled egg and steamed egg hotchpotch; dairy products such as cheese and yogurt; fish meat; animal meat; fruit; vegetable; and their frozen foods; refrigerated foods; chilled foods; retort-pouch foods; dried foods; freeze-dried foods; bottled vegetables; canned foods; premixes for pudding, pancake and butter; instant foods such as instant *"shiruko"* (sweet bean paste soup) and instant soup; baby food; therapeutic diet; peptide food; liquors such as refined *"sake"* (rice wine), blended liquor, liqueur, western liquor, beer and sparkling liquor; soft drinks such as Japanese tea, tea, coffee, hot chocolate, juice, carbonated drink, lactic acid drink, and lactic acid bacteria drink. Also they are usable as medicines or quasi-drugs in the form of a solid (tablet, powder or granule), paste, syrup or liquid.

The present invention is explained by the following experiments.

### Experiment 1

### Preparation of equol glycoside

Since S-equol is known to have higher physiological activity than R-equol, S-squol glycoside was prepared as follows. According to the method described in Example 1 of Japanese Patent Kohyo No. 504409/2006, 2 g of commercially available equol (a mixture of S-equol and R-equol commercialized by Funakoshi Co., Ltd.) was applied on "CHIRALCEL OJ-H" (a chiral column for separating stereoisomers, 4.6 mm ID × 250 mm, commercialized by Daicel Chemical Industries, Ltd.), eluted with mixture of solution-A (hexan: ethanol = 9:1) and solution-B (hexane: ethanol =1:9) as mobile phase, by liner gradient of 0% to 100% solution-B for 15 minutes at a flow rate of 1 ml/min, and then S-equol and R-equol were obtained separately. After 0.7 g of the obtained S-equol was dissolved in 70 ml of ethanol, the solution was added to 700 ml of a solution, containing 70 g of "PINEDEX #1" (a dextrin product commercialized by Matsutani Chemical Industries, Co., Ltd.), 50 mM acetate buffer pH 6.0 and 0.2 mM calcium chloride. The solution was admixed with 1,000 U/g of CGTase from *Bacillus stearothermophillus* (produced by Hayashibara Biochemical Laboratories, Inc.) and incubated at 40°C for 72 hours to give a transglycosylated reaction mixture. After heating at 100 °C to terminate the reaction, the solution was admixed with 70 ml of 1 M acetate buffer and 10 U/g of glucoamylase (produced by Seikagaku Corporation), and incubated at 40°C for 18 hours. The resultant reaction mixture was applied to a column (30 mm ID × 900 mm, 640 ml) packed with "DIAION HP-20" (a synthetic porous adsorption resin commercialized by Mitsubishi Chemical Industries Limited). After saccharides were eluted off with water, 10%, 20%, 30%, 40%, 50%, 60% and 100% ethanol were sequentially flowed, and equol glycosides in 40% ethanol eluent were collected. The collected eluent was evaporated to remove ethanol for concentration. The resultant solution containing the reaction products was subjected to preparative high performance liquid chromatography (hereinafter, abbreviated as "HPLC") using D-ODS-5 column, to which about 30 mg of the dry solid was applied per one batch. The reaction products were eluted by using an acetonitrile/water/acetic acid (= 18:82:0.1) elution solvent, and two fractions containing different reaction products were separately collected by monitoring UV absorbance. The fraction containing either of the reaction products was lyophilized after evaporated to remove the solvent for concentration. A portion of each of the lyophilized products was subjected to analytical HPLC and the eluent was analyzed by liquid chromatography-mass spectrometry (hereinafter, abbreviated as "LC/MS"). The analysis was carried out under the conditions described as follows:

### Preparative HPLC

Device: CR-4A and LCD-10AD (commercialized by Shimadzu Corporation)
Column: D-ODS-5 S-5 20 mm ID × 250 mm (commercialized by YMC Co., Ltd)
Mobile phase: acetonitrile/water/acetic acid (= 18:82:0.1)
Flow rate: 6 ml/min
Temperature: 40°C
Detection: UV absorbance

### Analytical HPLC

Device: CR-4A and LCD-10AD (commercialized by Shimadzu Corporation)
Column: ODS-AM-303 4.6 mm ID × 250 mm (commercialized by YMC Co., Ltd)
Mobile phase:
   Solution-A (acetonitrile/water/acetic acid (= 20:80:0.1))
   Solution-B (acetonitrile/water/acetic acid (= 40:60:0.1))
   programmed to flow Solution-A alone for initial 25 minutes, 0% to 100% solution-B by liner gradient for next 10 minutes, and Solution-B alone for last 10 minutes
Flow rate: 0.8 ml/min
Temperature: 35°C
Detection: UV absorbance

### UV detection

Device: SPD-10AVP (Shimadzu Corporation)
Wave length: 280 nm

### LC/MS

Device: LCQ Advantage (Thermoelectron Co., Ltd.)
Analytical conditions:
   ESI, negative mode, m/z 50 - 2000
   Sheath gas flow: 60
   Ion spray voltage: 6 kV
   Capillary temperature: 240 °C
   Capillary voltage: -8.0V
   Tube lens offset: -10.0 V

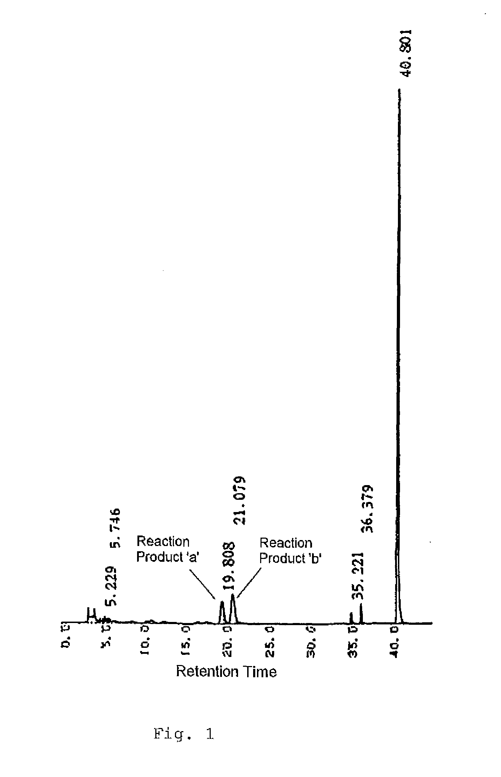

When the reaction mixture after glucoamylase treatment was subjected to analytical HPLC, peaks of two reaction products having absorbance at UV 280 nm (having equol structure) appeared at retention times of about 19 minutes and about 21 minutes as shown in Fig 1 (hereinafter, these products were described as "reaction product 'a'" and 'b', respectively). The yields of reaction product 'a' and 'b' were respectively calculated to be 70.1 mg and 92.4 mg, based on the peak area of these products in the elution chart detected in UV 280 based on molar absorption coefficient of equol (regarding equol and equol glycosides have the same molar absorption coefficient because glycosyl group has no UV absorbance).

After the reaction mixture was subjected to DIAION HP-20 column chromatography, 40% ethanol eluent containing equol glycosides was collected and concentrated to obtain a reaction product solution. The purity of the glycosides in the solution, on a dry solid basis, was 41.4% and the yields of reaction products 'a' and 'b' were 57.1 mg and 79.0 mg, respectively. Equol was eluted in 50% to 100% ethanol fractions.

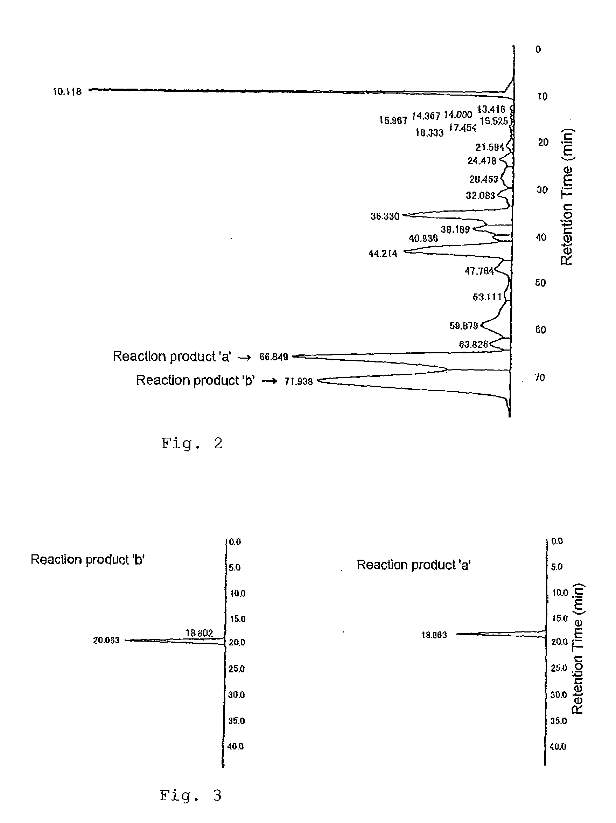

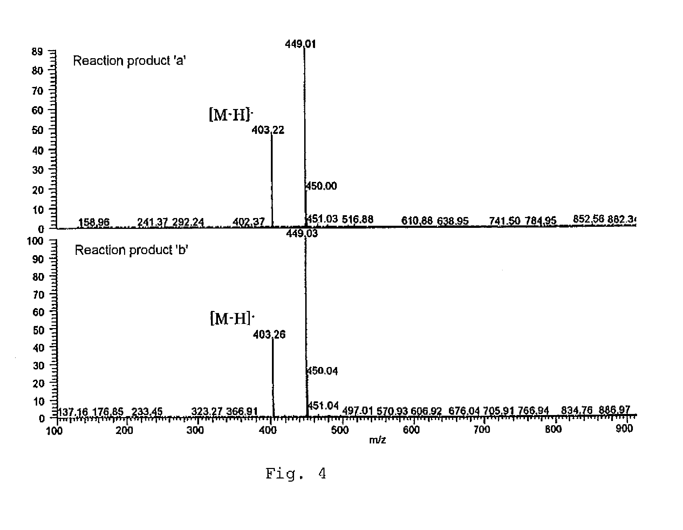

Fig 2 shows an elution profile of the reaction product solution on the preparative HPLC, in which about 30 mg of dry solid was applied per onebatch. The fractions of reactionproduct 'a' and 'b' were collected by monitoring the UV absorbance. The yields of reaction products 'a' and 'b' were respectively 45.3 mg and 60.3 mg, and the purity thereof were calculated to be about 100% and 98.8%, respectively, by the analytical HPLC. As evident from Fig. 4, which shows the ion intensity vs. M/Z (ration of molecular mass and charge) in MS analysis of these two peaks, an ion having mass number of 403, formed by elimination of one hydrogen atom, was observed in each peak, revealing that the molecular masses of both reaction products were 404.

Using 15 mg of either of the reaction products 'a' and 'b' , dissolved in 0.7 ml of methanol-*d*₄, NMR analysis was carried out to determine their structures under the following conditions.

### NMR measurement

Device: JNM-AL-300 (JEOL Limited)
Solvent: D₂O
Standard: Sodium trimethylsilylpropionic acid-2,2,3,3- *d*₄ Magnetic field intensity:
   ¹H-NMR: 300.4 MHz
   ¹³C-NMR: 75.45 MHz
Integration number of measurement:
   ¹H-NMR: 8
   ¹³C-NMR: 1000
   COSY: 4
   C-H COSY: 120

### NMR analysis

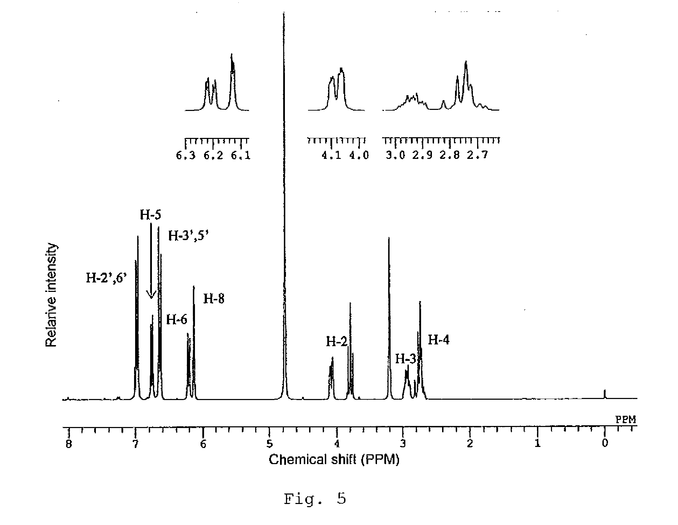

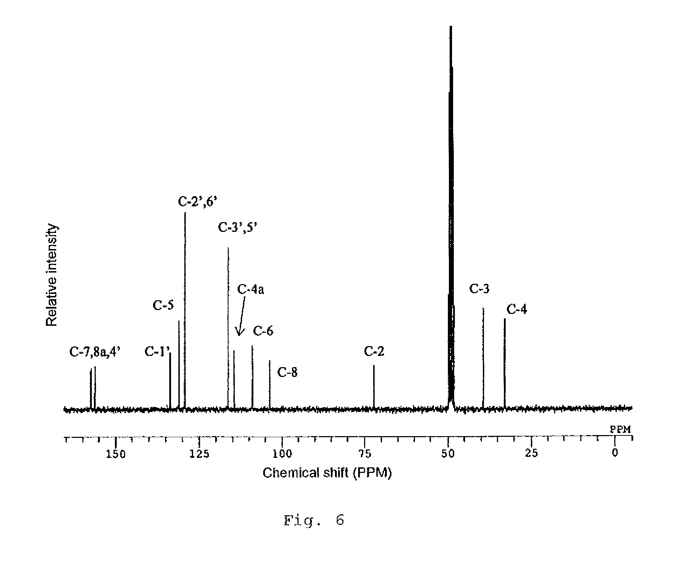

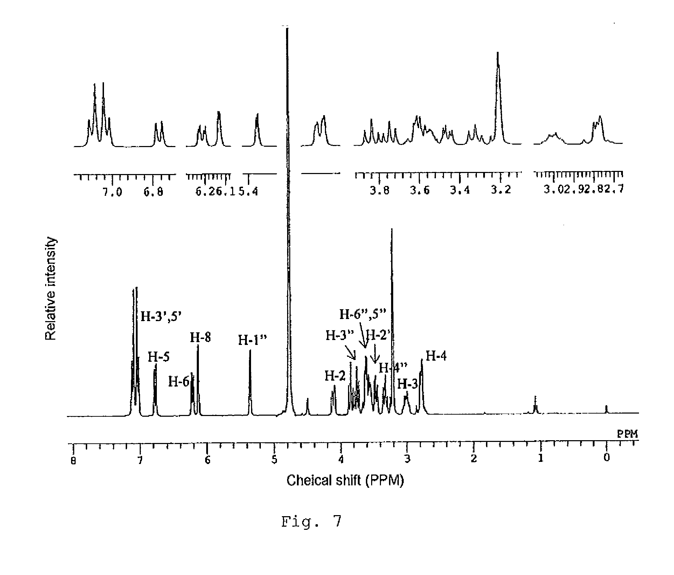

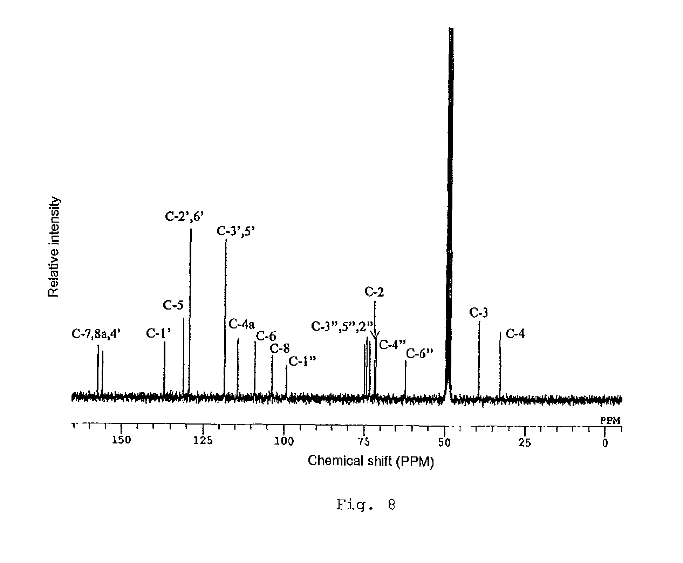

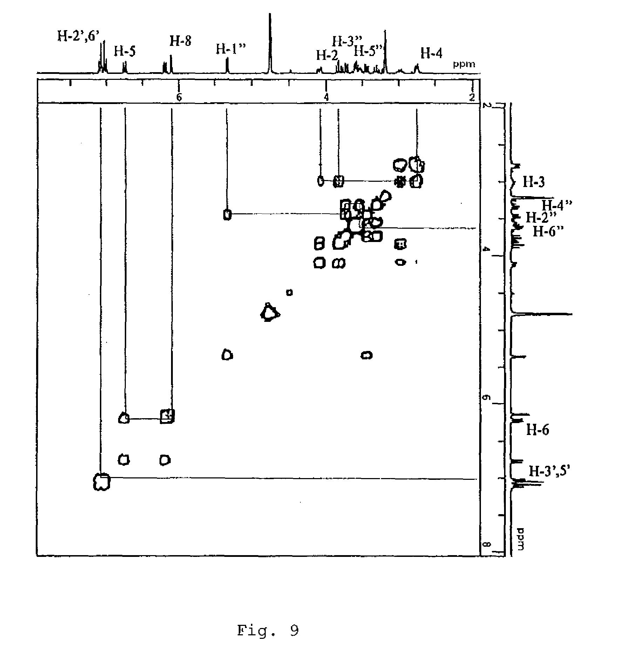

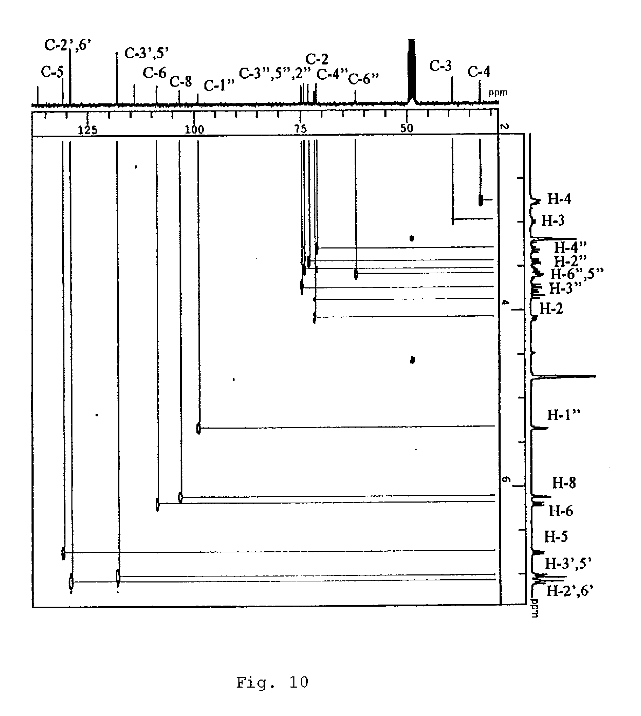

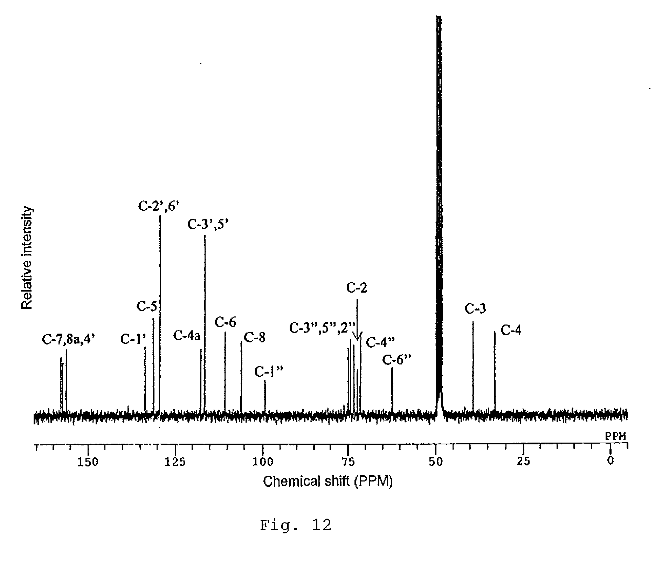

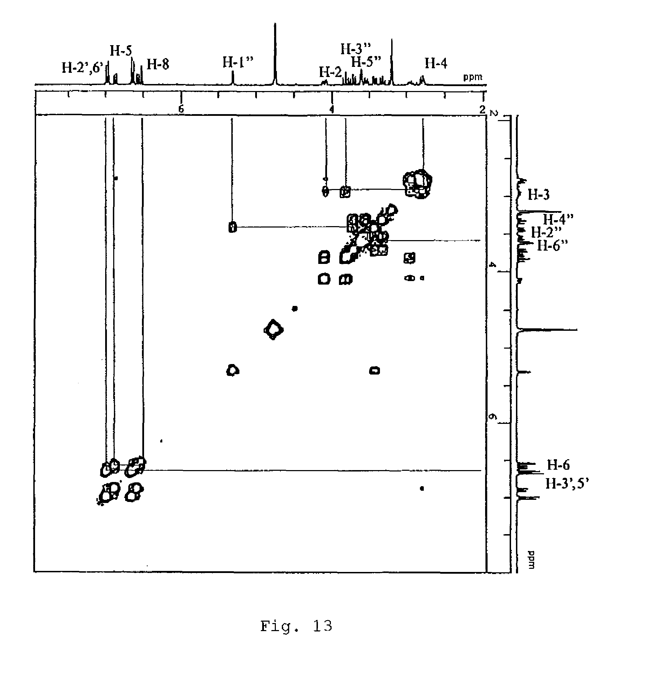

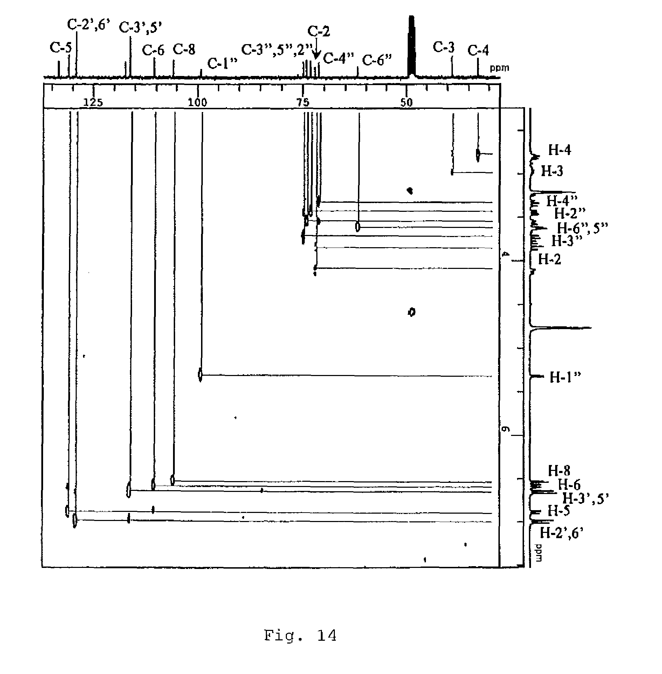

Using the chemical shift data of equol obtained by NMR analysis (shown in Fig. 5 and 6) as reference, all NMR signals of the reaction products 'a' and 'b' were respectively assigned according to the chemical shift data of NMR analysis (shown in Figs. 7 to 14). The result is in Table 1.

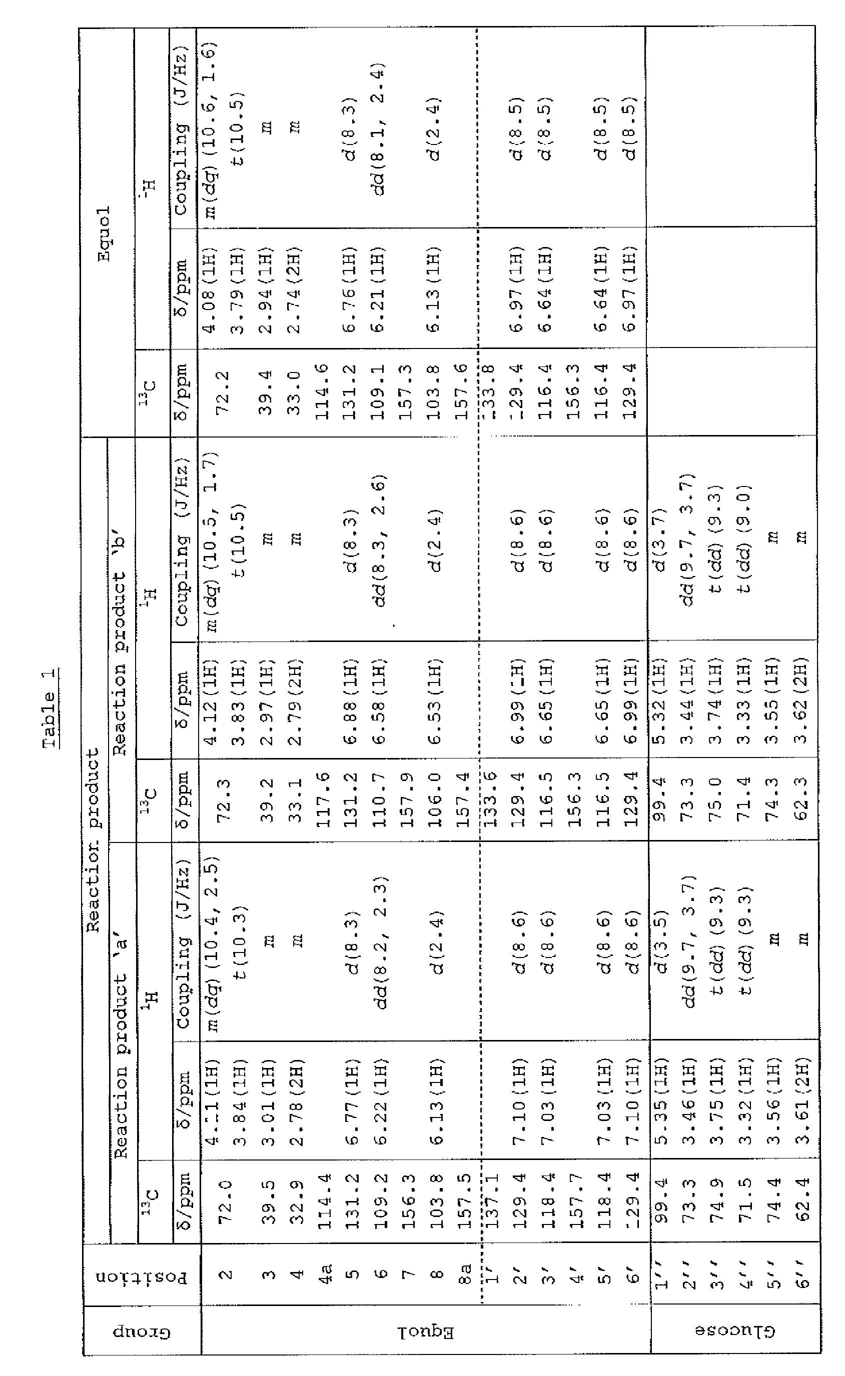

### Equol

¹H- and ¹³C-NMR spectra of equol are shown in Figs. 5 and 6, respectively. As shown in Table 1, signals of *p*-disubstituted benzene at 6.97 ppm and 6.64 ppm and signals of 1,2,4-tri-substituted benzen at 6.76 ppm, 6.21 ppm and 6.13 ppm were observed in ¹H-NMR. It is considered that the formers were assigned to H-2' or H-6' and H-3' or H-5' of B-ring of S-equol, respectively, and the latters were assigned to H-5, H-6 and H-8 of A-ring of S-equol, respectively. The chemical shifts (δ) of alkyl protons and carbons in A-ring of S-equol were considered to be as follows: H-2, 4.08 ppm and 3.79 ppm; C-2, 72.2 ppm; H-3, 2.94 ppm; C-3, 39.4 ppm; H-4, 2.74 ppm; and C-4, 33.0 ppm.

### Reaction product 'a'

The NMR spectra are shown in Figs. 7 to 10. As shown in Table 1, in regard to B-ring of equol represented in Chemical Formula 1, the signals of protons observed in ¹H-NMR and the signals of C-1', C-3' and C-5' observed in ¹³C-NMR shifted to lower magnetic regions compared to those of S-equol described above. The chemical sifts of the glucosyl group indicated that the structure is pyranose-form of OH-1 binding via α-linkage (H-1, 5.35 ppm (J = 3.5 Hz); C-1, 99.4 ppm). These results indicate that the reaction product 'a' is 4'-O-α-D-glucopyranosyl-S-equol in which a glucosyl group binds to OH-4' of B-ring of S-equol (represented by Chemical Formula 2).

### Reaction product 'b'

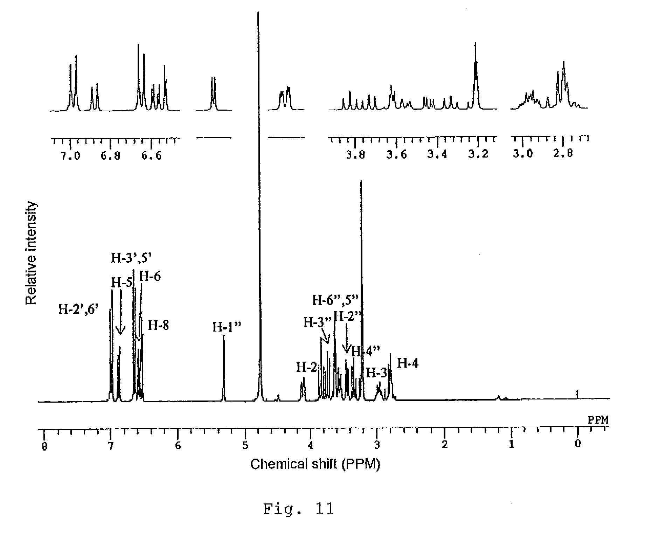

The NMR spectra are shown in Figs. 11 to 14. As shown in Table 1, in regard to A-ring of equol represented in Chemical Formula 1, the signals of protons observed in ¹H-NMR and the signals of C-4a, C-6 and C-8 observed in ¹³C-NMR shifted to lower magnetic regions compared to those of S-equol described above. The chemical sifts of the glucosyl group indicated that the structure is pyranose-form of OH-1 binding via α-linkage (H-1, 5.32 ppm (J = 3.7 Hz); C-1, 99.4 ppm). These results indicate that reaction product 'b' is 7-O-α-D-glucopyranosyl-S-equol in which a glucosyl group binds to OH-7 of A-ring of S-equol (represented by Chemical Formula 3).

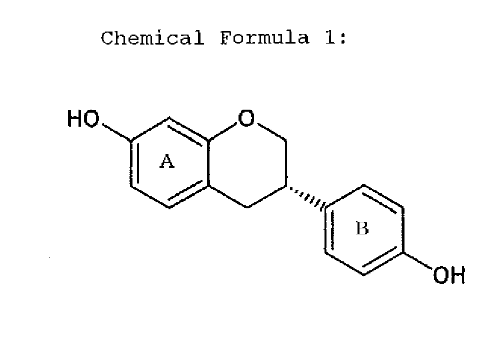

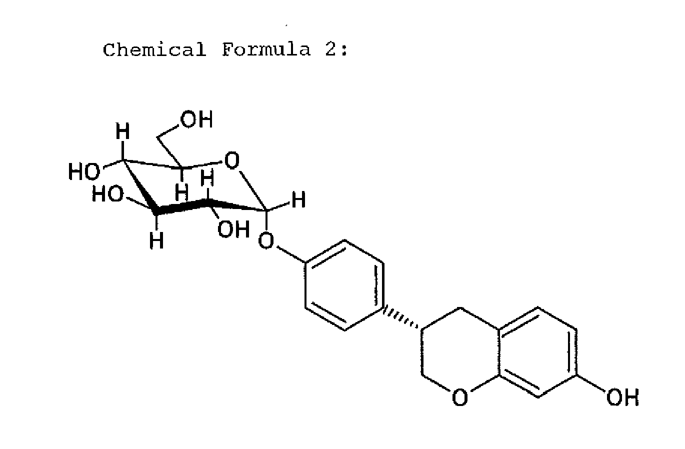

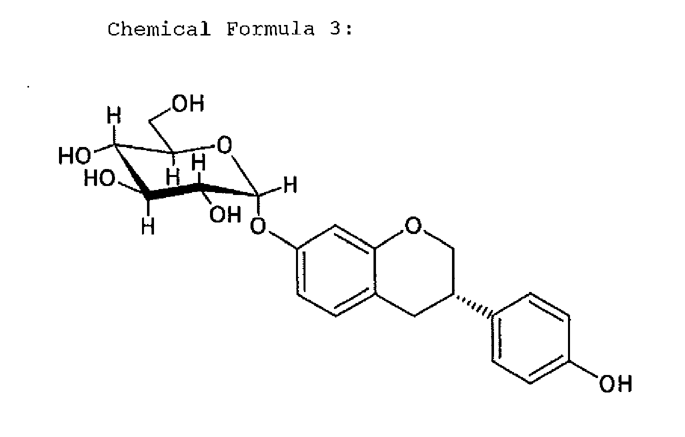

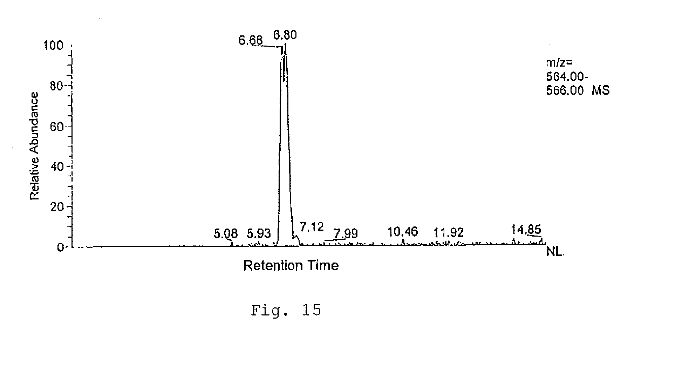

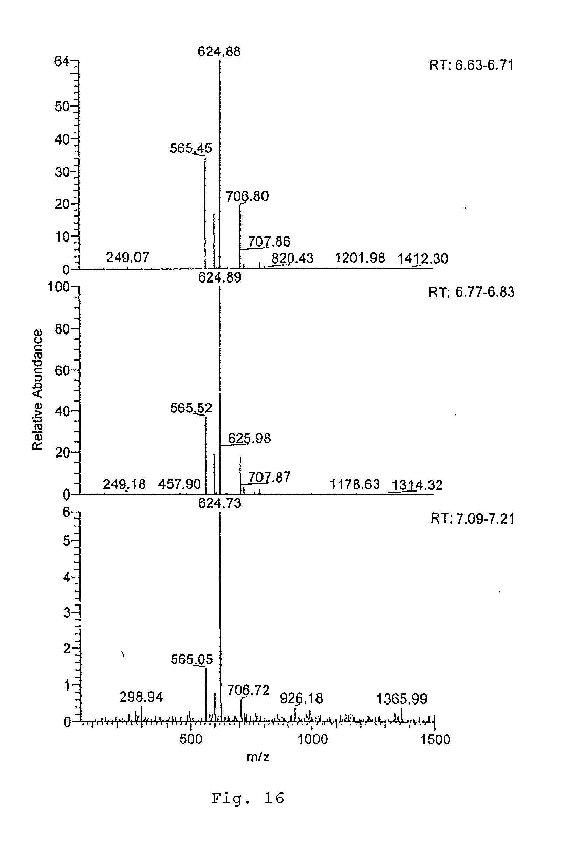

When the fraction containing equol glycosides obtained by HP-20 column chromatography was subjected to analytical HPLC using ODS-AM-303 column and the eluent was analyzed by LC/MS, three peaks were observed in the range of M/Z 564 to 566 at retention times of 6.63 to 6.71 minutes, 6.77 to 6.83 minutes, and 7.09 to 7.21 minutes (Fig. 15). Since each fraction had UV absorption feature at 280 nm and ion having molecular mass of 565 was detected in each fraction (Fig.16), it was revealed that these fractions contain three reaction products having molecular mass of 566 with different hydrophobicity. Since molecular mass of these reaction products correspond to that of equol glycoside in which two glucose residues bind to equol and equol has only two OH groups (4' and 7) at which glucosyl residue can bind, these three products of molecular mass of 566 were considered to be 4'-O-α-D-maltosyl-S-equol in which maltosyl group bind to OH-4' of equol via α-linkage, 7-O-α-D-maltosyl-S-equol in which maltosyl group bind to OH-7 of equol via α-linkage, remained even after glucoamylase treatment; and 4',7-O-α-di-D-glucopyranosyl-S-equol in which one glucosyl residue bind to each of OH-4' and OH-7 of equol via α-linkage. These results indicate that an equol glycoside having glucosyl group on both OH groups of S-equol is also formed by transglycosylation with cyclomaltodextrin glucanotransferase (CGTase), even though the production yield is lower than those of equol glycosides having glucosyl group on either of the OH groups. The HPLC analysis using column ODS-AM-303 was carried out in the same condition of the analytical HPLC described above except for using the mobile phase and flow rate described below.

### Mobile phase

Solution-A: Acetonitrile/water/acetic acid (= 15:85:0.1)
Solution-B: Acetonitrile/water/acetic acid (= 35:65:0.1)
Flow rate: 1 ml/min with liner gradient of 50% to 100% solution-B in 40 minutes

### Experiment 2

### Test of whitening effect in vitro

Three equol glycoside preparation prepared in Experiment 1, i.e. the solution containing reaction products (a solution containing 4'-O-α-D-glucopyranosyl-S-equol and 7-O-α-D-glucopyranosyl-S-equol, hereinafter, described as "equol glycoside containing solution"), 4'-O-α-D-glucopyranosyl-S-equol and 7-O-α-D-glucopyranosyl-S-equol, and the equol used in Experiment 1 were used as test samples, and the following experiment was carried out to determine their whitening effect. Mouse B16 melanoma cells suspended in RPMI-1640 medium containing 10v/v % fetal calf serum (hereinafter, abbreviated as "FCS") were inoculated into "6-MULTIWELL MICROPLATE" (6-well microplate commercialized by Becton, Dickinson and Company) to give a cell concentration of 4 × 10⁴ cells/well. After the cells were adhered to the bottom of well, the culture supernatant was removed. The cells were admixed with equol or any one of the three equol glycoside samples, diluted witch the same medium to give the concentration described in Table 2, and then cultured at 37°C in an atmosphere of 5 v/v % CO₂ for four days. The cells were collected after admixed with trypsin-EDTA, washed once with Dulbecco's PBS (-) (hereinafter, abbreviated as "D-PBS"), and then dissolved in 1N-sodium hydroxide. A part of the solution was used to determine the amount of protein. The above alkaline solution was boiled for 30 minutes and the amount of melanin was measured. Table 2 shows the amount of melanin per 1 mg of protein, which was calculated by dividing the amount of melanin in B16 melanoma cells cultured with each test sample, with the total amount of protein in the cell. Also ED50 (the concentration of the test sample giving half of the melanin production in the cell cultured with negative control) is shown in Table 2. The medium alone was used as negative control, and koji acid, which has been used as a whitening substance for external dermatological composition, was used as positive control at the concentration described in Table 2 (0.5 to 2 mM), to evaluate the whitening effect. The evaluation of whitening effect and measurement of melanin, protein and equol glycosides were carried out as follows.

### Criteria of whitening effect

Compared to the browning observed when the medium without test samples was used, the following symbols mean;
-: Not changed;
±: Slightly whitened;
+: A little whitened;
++: Significantly whitened;
+++: Completely whitened.

### Measurement of melanin

The melanin production was determined by comparing the absorbance at 450 nm (reference 650 nm) of each alkaline solution to that of melanin standard (Sigma Corporation), measured with "MICROPLATE READER M-Vmax" (commercialized by Wako Pure Chemical Industries Co., Ltd).

### Measurement of protein

The amount of protein was determined by Bradford method using human serum albumin as standard.

### Measurement of equol glycoside

The concentration of equol glycoside in the powdery product used in the following experiments were determined by the same method as used in Experiment 1, based on the molar absorption coefficient of equol measured at UV 280 nm.

**Table 2**

| Test Sample | Sample concentration | Determination of whitening effect | Melanin Production (µg/mg-cell protein) | ED50 |
|---|---|---|---|---|
| Negative control | 0 | - | 73.9 | - |
| Positive control (Koji cid) | 1 mM | + | 57.1 | 4.0 mM |
| | 2 mM | ++ | 47.5 | |
| | 4 mM | +++ | 37.7 | |
| Equol | 5 µM | ++ | 36.8 | 4.1 µM |
| | 10 µM | +++ | 30.7 | |
| | 20 µM | +++ | 29.8 | |
| Solution containing equol glycosides | 5 µM | + | 58.1 | 40.4 µM |
| | 10 µM | + | 57.0 | |
| | 20 µM | ++ | 49.2 | |
| 4-O-α-glucosyl-S-equol | 5 µM | + | 57.1 | 38.8 µM |
| | 10 µM | + | 55.0 | |
| | 20 µM | ++ | 48.2 | |
| 7'-O-α-glucosyl-S-equol | 5 µM | + | 54.8 | 27.3 µM |
| | 10 µM | + | 48.4 | |
| | 20 µM | ++ | 43.0 | |

As is evident from Table 2, the test samples of equol exerted whitening effect (inhibitory effect on melanin production) on B16 melanoma cell depending on the concentration in the range of 5 µM to 20 µM, and the efficiency was approximately the same as that of 1 mM koji acid as positive control. Every one of three equol glycosides exerted whitening effect (inhibitory effect on melanin production) on B16 melanoma cell in the range of 5 µM to 20 µM as equol. Among the three equol glycosides, 7-O-µ-D-glucopyranosyl-S-equol exerted the effect at a relatively lower concentration than the others in terms of ED50, however, the effect visually evaluated was not significantly different.

These experimental results indicate that external dermatological agent incorporated with equol and/or its glycosides exert excellent effect of whitening and/or beautifying the skin and antiaging because they have inhibitory effect on melanin production.

### Experiment 3

### Effect of equol glycoside on lipocyte

The following experiment was carried out to investigate the effect of equol glycoside on lypocite. Lipocyte, having high sensitivity to insulin, ingests serum saccharide and accumulates it in the form of triglyceride in the cell. Since insulin regulates the serum saccharide level by inducing differentiation of precursor lipocyte into lipocyte, the effect of equol glycoside was investigated on differentiation of precursor lipocyte into lipocyte and on intracellular lipid in the lipocyte.

### Effect of equol glycoside on differentiation into lipocyte

It is known that mouse 3T3-L1 cell line (precursor lipocyte) differentiates into mature lipocyte when cultured in the presence of a differentiation inducer, and accumulates oil droplet in the cell according to the progress of differentiation. The mouse 3T3-Li cells were suspended in a medium, which is Dulbecco's MEM medium (hereinafter, abbreviated as "D-MEM") supplemented with FCS (final concentration of 10 v/v %) and glucose (final concentration of 3 mg/ml), (hereinafter, described as "regular medium") to give a cell concentration of 2.5 × 10⁴ cells/well, and inoculated to "CELL CULTURE 24-MULTIWELL MICROPLATE" (24-well microplate coated with collagen type 1, commercialized by Becton, Dickinson and Company) by 1ml/well. After the cells were cultured at 37°C in an atmosphere of 5 v/v % CO₂ for two days, the supernatant was removed and the remaining cells were admixed with a medium containing 10 µg/ml insulin and equol or equol glycosides dissolved in 50% ethanol, to give a concentration in Table 3, by 1ml/well and then cultured for 12 days while replacing the medium every two or three days. After each well of the plate was washed with "D-PBS" three times, D-PBS containing 10% formalin was added by 0.2 ml/well, and then the cell was placed at 4°C for one hour for fixation. After the fixing fluid was removed, a staining fluid of oilred O was added by 0.15 ml/well, and allowed to react at ambient temperature for 15 minutes to stain the lipid droplet in 3T3-L1 cell. After the staining fluid was removed, each well was washed with distilled water three times, and then the plate was dried. After the pigment in each well was dissolved in 0.4 ml of isopropanol, the absorbance of the resulting solution was measured at 490. The progress of the cell differentiation was expressed with relative absorbance to the absrobance of the cell cultured with control regarded as 100%. The results are in Table 3.

### Test for lipid hydrolysis

After the culture supernatant of mouse 3T3-L1 cells cultured under the same condition described above was removed, a regular medium containing 5 µg/ml insulin, 0.25 µM dexamethasone and 0.5 mM 3-isobutyl-1-methylxanthin was added by 1 ml/well, and then the cells were cultured for two days to differentiate into mature cells. After the supernatant was removed, the cells were washed with D-PBS three times and cultured with regular medium for 12 days while replacing the medium every two or three days by 1ml/well. After the supernatant was removed and the cells were washed with D-PBS two times, a solution containing equol or equol glycosides diluted with D-MEM containing 1% bovine serum albumin to give the concentration described in Table 4 was added by 0.25 ml/well, and then the cell was cultured for 6 hours. After the supernatant was collected, the amount of glycerol was measured to determine the amount of the degraded lipid. After the supernatant was removed and the cells were washed with D-PBS three times, D-PBS containing 0.25% Triton X-100 was added to collect the cells, and then the cells were sonicated to give a cell-dissolving solution. The amount of protein of the sonicated cells was measured by Bradford method as in Experiment 3, and the amount of glycerol per cell protein representing degree of lipid degradation was shown in Table 3. The amount of glycerol was measured with "GLYCEROL REGENT A" (glycerol measurement kit commercialized by Zen-Bio, Inc.).

**Table 3**

| Test Sample | Sample concentration | Enhancing effect on differentiation into lipocyte (%) | Enhancing effect on lipid hydrolysis (µg/mg-cell protein) |
|---|---|---|---|
| Control (0.22% ethanol) | 0 | 100 | 274 |
| Equol | 5 µM | 194* | 392* |
| | 20 µM | 430* | 679* |
| | 80 µM | ND | 1119* |
| Powdery product containing equol glycosides | 5 µM | 95 | 282 |
| | 20 µM | 103 | 335* |
| | 80 µM | 255* | 421* |
| 4'-O-α-glucosyl-S-equol | 5 µM | 92 | 272 |
| | 20 µM | 107 | 320* |
| | 80 µM | 217* | 401* |
| 7-O-α-glucosyl-S-equol | 5 µM | 96 | 265 |
| | 20 µM | 101 | 312* |
| | 80 µM | 253* | 374* |

| | | | |
|---|---|---|---|
| *:Significantly different from control (p < 0.05) | | | |

As is evident from Table 3, equol at a concentration of 5 µM or higher enhanced the degradation of triglyceride accumulated in lipocyte as well as enhancing the differentiation of precursor lipocyte (3T3-11) into mature lipocyte, compared to control. Every equol glycoside sample of powdery product containing equol glycosides, 4'-O-α-D-glucopyranosyl-S-equol and 7-O-α-D-glucopyranosyl-S-equol differentiated 3T3-L1 cell into mature lipocyte when added at a concentration of 80 µM, and enhanced the degradation of triglyceride accumulated in lipocyte when added at a concentration of 20 µM or higher.

There results indicate that since equol and its glycosides have activity of inducing differentiation of precursor lipocyte into mature lipocyte and of degrading triglyderide accumulated in lipocyte, equol and/or its glycosides have preventive, improving and therapeutic effect on life-style related diseases such as diabetes and metabolic syndrome by regulating lipid metabolism.

The present invention is explained by the following examples, but should not be restricted by them.

### Example 1

### Preparation of equol glycoside

After one gram of a regent grade equol (a mixture of R-equol and S-equol, product No. 26355-54 commercialized by Nacalai Tesque, Inc.) was dissolved in a solvent into 100 ml solution, the solution was added to 1,000 ml of a solution containing 100 g of "PINEDEX #1" (dextrin commercialized by Matsutani Chemical Industries Co., Ltd.), 50 mM acetate buffer pH 6.0 and 2 mM calcium chloride. The solution was admixed with CGTase from *Bacillus stearothermophillus* (produced by Hayashibara Biochemical Laboratories, Inc.) by 1,000 U/g and incubated at 40°C for 72 hours. After the reaction was terminated by heating at 100°C, the reaction mixture was admixed with 70 ml of 1 M acetate buffer and glucoamylase (Seikagaku Corporation) by 10 U/g and incubated at 40°C for 18 hours. The reaction solution was applied to a column (3 cm ID × 90cm, 640 ml) packed with "DIAION HP-20" (synthetic porous adsorption resin commercialized by Mitsubishi Chemical Industries Limited). After saccharides were washed out with water from the column, equol glycosides were sequentially eluted with 30% and 40% ethanol, and 40% ethanol fraction containing equol glycosides was collected. After the fraction was evaporated to remove ethanol for concentration, the resultant solution was lyophilized to obtain a powdery product containing equol glycosides.

The powdery product or the preparation before purified with DIAION HP-20, in any form of preparation without modification, purified preparation, preparation fractionated into each component, or pulverized preparation, is usable as a whitening agent and/or lipid metabolism improving agent, or as a material in a composition such as cosmetics, medicine, quasi-drug, and foods and beverages. Since equol glycosides also have estrogenic, antioxidant and antiallergic activities, the composition containing equol glycosides is usable for beautifying the skin, preventing or improving menopausal symptoms or osteoporosis, preventing or treating skin-aging orwrinkle, palliating allergic symptoms, preventing and palliating obesity, preventing and palliating diabetes, and for preventing or treating cardiac or vascular diseases, and various kids of disorders or symptoms caused by acute decrease of estrogen in ovaries.

### Example 2

### Preparation of R-equol glycoside

Each of S-equol and R-equol was separated from regent grade equol (a mixture of R-equol and S-equol commercialized by Funakoshi Co., Ltd.) by the same method described in Experiment 1. One gram of the resultant R-equol was dissolved in 100 ml of ethanol, the solution was added to 1000 ml of a solution containing 100 g of "PINEDEX #1" (dextrin commercialized by Matsutani Chemical Industries Co, . Ltd.), 50 mM acetate buffer pH6.0 and 2 mM calcium chloride. The solution was admixed with CGTase from *Bacillus stearothermophillus* (produced by Hayashibara Biochemical Laboratories, Inc.) by 1,000 U/g and incubated at 40°C for 72 hours. After the reaction was terminated by heating at 100°C, the reaction mixture was admixed with 100 ml of 1 M acetate buffer and glucoamylase (produced by Seikagaku Corporation) by 10 U/g and incubated at 40°C for 18 hours. The reaction solution was applied to a column (3 cm ID × 90cm, 640 ml) packed with "DIAION HP-20" (synthetic porous adsorption resin commercialized by Mitsubishi Chemical Industries Limited). After saccharides were washed out with water from the column, equol glycoside was sequentially eluted with 30% and 40% ethanol, and then 40% ethanol fraction containing equol glycosides was collected. After the fraction was evaporated to remove ethanol and concentrated, the solution was lyophilized to give a powdery product of equol glycosides.

The powdery product or the preparation before or after purified with DIAION HP-20, in any form of preparation without modification, purified preparation, or mixture of each component, are usable as whitening agent and/or lipid metabolism improving agent or as a material in a composition such as cosmetics, medicine, quasi-drug and foods and beverages. Since equol glycosides also have estrogenic, antioxidant and antiallergic activities, the equol glycosides or the composition containing them can be used for beautifying skin, for preventing or improving menopausal symptoms or osteoporosis, for preventing or treating skin-aging or wrinkle, for palliating allergic symptoms, for preventing and palliating obesity, for preventing and palliating diabetes, for preventing or treating cardiac or vascular diseases, and various kids of disorder or symptoms caused by acute decrease of estrogen in ovaries.

The powdery product was dissolved in 50% ethanol, subjected to preparative HPLC using D-ODS-5 S-5 column by the same method as in Experiment 1 and the eluent was analyzed by LC/MS. In the LC/MS, two peaks of substances having UV absorption at 280 nm (having equol structure) were detected at the same retention time as 4'-O-α-D-glucopyranosyl-S-equol or 7-O-α-D-glucopyranosyl-S-equol prepared in Experiment 1. In MS analysis of them, ion of molecular mass of 403 was detected in each peak as S-equol glycoside in Experiment 1, indicating that molecular mass of both reaction products is 404. Three peaks of ions having molecular mass of 565 were also detected at almost the same retention time as the peaks having molecular mass of 565 detected in S-equol in Experiment 1.

These results indicate that when cyclomaltodextrin glucanotransferase (CGTase) is allowed to act on a mixture of R-equol and dextrin, R-equol glycoside is formed. Since three molecules having molecular mass equal to equol binding to two glucose, it is considered that when CGTase is allowed to act on a mixture of R-equol represented in Chemical Formula 5 and dextrin, 4',7-O-α-di-D-glucopyranosyl-R-equol in which one glucosyl group binds to each one of two HO groups via α-linkage was formed as well as 4'-O-α-D-glucopyranosyl-R-equol and 7-O-α-D-glucopyranosyl-R-equol, in which one glucosyl group binds to either one of two OH groups via α-linkage.

The yields of 4'-O-α-D-glucopyranosyl-R-equol and 7-O-α-D-glucopyranosyl-R-equol by the preparative HPLC using D-ODS-5 S-5 column were 38.9 mg and 50.3 mg, respectively, and their purity were 99.6% and 98.4%, respectively.

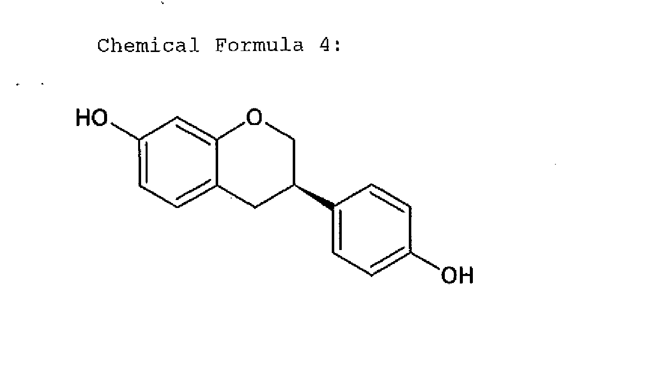

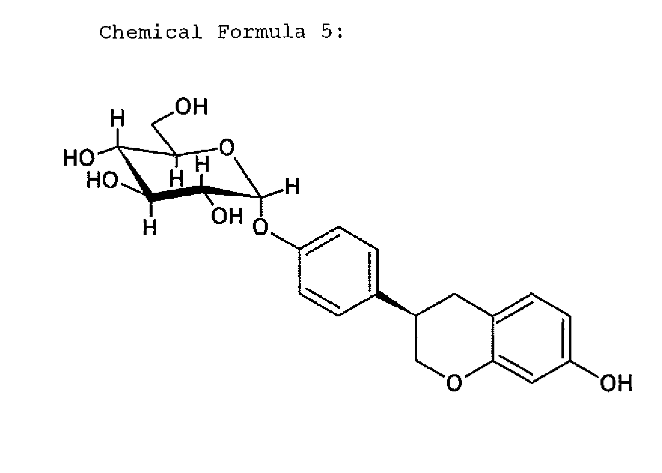

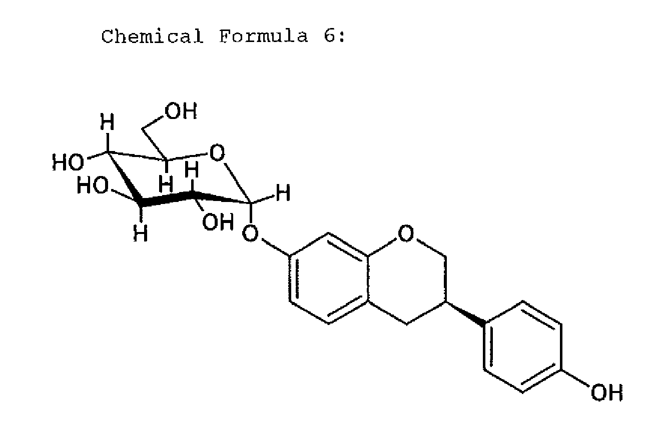

These purified R-equol are usable as whitening agent and/or lipid metabolism improving agent by itself or as a material contained in a composition such as cosmetics, medicine, quasi-drug and foods and beverages. Since equol glycosides also have estrogenic, antioxidant and antiallergic activities, the equol glycosides isolated or purified by the preparative HPLC or the composition containing them can be used for beautifying skin, for preventing or improving menopausal symptoms or osteoporosis, for preventing or treating skin-aging or wrinkle, for palliating allergic symptoms, for preventing and palliating obesity, for preventing and palliating diabetes, for preventing or treating cardiac or vascular diseases, and various kids of disorder or symptoms caused by acute decrease of estrogen in ovaries.

### Safety test

Three parts (in equol equivalent) by weight of either of the lyophilized powdery products of DIAION HP-20 column chromatography eluent containing equol glycosides prepared in Example 1, 4'-O-α-D-glucopyranosyl-S-equol or 7-O-α-D-glucopyranosyl-S-equol prepared by D-ODS-5 S-5 column chromatography in Experiment 1, or 4'-O-α-D-glucopyranosyl-R-equol or 7-O-α-D-glucopyranosyl-R-equol prepared by D-ODS-5 S-5 column chromatography in Experiment 2 was dissolved in a base solution containing five parts by weight of ethanol, five parts by weight of glycerol and 87 parts by weight of water to give a test solution. Each of the test solution was applied on the upper arm of five volunteers (the total number of volunteers is 25) in an area of 1-cm square three times a day for three days, then the condition of the skin applied with the test solution was observed for the next week. As control, the base solution alone was applied on an area of 1-cm square adjacent to the area applied with the test solution three times a day for three days, and then the condition of the skin applied with the control solution was observed for one week from the initial application. Since the application of either of the test solution or control caused no rough dry skin or rubor by application of, these three equol glycosides was considered to be highly safe materials for an external dermatological composition.

### Example 3

### Preparation of S-equol glycosides

4'-O-α-D-Glucopyranosyl-S-equol and 7-O-α-D-glucopyranosyl-S-equol were prepared and purified from 1 g of S-equol, isolated by separating from R-equol in Example 2, by the method in Experiment 1. Their yields were 40.2 mg and 53.6 mg, respectively, and their purity were 99.5% and 98.5%, respectively.

The above products, the preparation before purified with DIAION HP-20, or the preparation after eluted from DIAION HP-20 are usable, in a form of the preparation without modification, purified preparation, or of mixture of the glycosides, as whitening agent and/or lipid metabolism improving agent or as a material contained in a composition such as cosmetics, medicine, quasi-drug and foods and beverages. Since equol glycosides also have estrogenic, antioxidant and antiallergic activities, the equol glycosides or the composition containing them can be used for beautifying skin, for preventing or improving menopausal symptoms or osteoporosis, for preventing or treating skin-aging or wrinkle, for palliating allergic symptoms, for preventing and palliating obesity, for preventing and palliating diabetes, for preventing or treating cardiac or vascular diseases, and various kids of disorder or symptoms caused by acute decrease of estrogen in ovaries.

### Example 4

### Powdery product containing equol glycosides

After the solution treated with CGTase in Example 1 was heated to inactivate the enzyme, the solution was desalted, deproteinized, and concentrated in conventional method. One part by weight of the resultant solution was admixed with one part by weight of "SANDEC #30" (maltodextrin produced by Sanwa Cornstarch Co., Ltd.), then spray-dried by conventional method to give a powdery product containing equol and equol glycosides.

The powdery product has low hygroscopicity and high preservation stability. The product is usable as whitening agent and/or lipid metabolism improving agent without modification, or as an ingredient in a composition such as cosmetics, medicine, quasi-drug and foods and beverages. Since equol and equol glycosides also have estrogenic, antioxidant and antiallergic activities, the composition containing equol glycosides can be used for beautifying skin, for preventing or improving menopausal symptoms orosteoporosis, for preventing or treating skin-aging or wrinkle, for palliating allergic symptoms, for preventing and palliating obesity, for preventing and palliating diabetes, for preventing or treating cardiac or vascular diseases, and various kids of disorder or symptoms caused by acute decrease of estrogen in ovaries.

### Example 5

### Whitening agent

Five tenth parts by weight of S-equol prepared by the method in Experiment 1 and one part by weight of sucrose fatty acid ester were dissolved in 8.5 parts by weight of water. Two parts by weight of trehalose was dissolved in ten parts by weight of the above resultant suspension of equol, and then the resultant solution was spray-dried to give a powdery product of S-equol. The product is usable as whitening agent and/or lipid metabolism improving agent without modification, or as an ingredient in a composition such as cosmetics, medicine, quasi-drug and foods and beverages. Since equol glycosides also have estrogenic, antioxidant and antiallergic activities, the equol glycosides or the composition containing them can be used for beautifying skin, for preventing or improving menopausal symptoms or osteoporosis, for preventing or treating skin-aging or wrinkle, for palliating allergic symptoms, for preventing and palliating obesity, for preventing and palliating diabetes, for preventing or treating cardiac or vascular diseases, and various kids of disorder or symptoms caused by acute decrease of estrogen in ovaries.

### Example 6

### Lotion

According to the formulation prescribed below, a lotion was prepared by conventional method. Hereinafter, the amount of the materials of the formulations in the following examples is represented as the mass ratio to the total mass of the product in percentage terms.

| | |
|---|---|
| (Formulation) | (%) |
| Glycerol | 5 |
| 1,3-Butyleneglycol | 6.5 |
| Polyoxyethylenesorbitan monolaurate (20 E.O.) | 1.2 |
| Ethyl alcohol | 8 |
| Powdery product containing equol glycoside | 0.5 (as equol) |
| prepared by the method in Example 1 | |
| Lactic acid | 0.05 |
| Sodium lactate | 0.1 |
| 2-Ethylhexyl p-methoxycinnamate | 0.1 |
| Pullulan | 0.05 |
| Antiseptic | Adequate |
| Flavor | Adequate |
| Purified water | Adequate |

The total amount was made into 100%.

The product was a beneficial cosmetic that exerted effect of whitening the skin and making the skin firm and glossy when applied to the skin. Since the product contained equol glycosides, it had high preservation stability.

The lotion was allowed to be used by ten volunteers tree times a day for 10 days, and then the a lotion containing equimolar equol in place of the equol glycoside was allowed to be used in the same method. In regard to their comfort, every volunteer answered that the lotion containing equol glycoside is less irritant than that containing equol.

### Example 7

### Lotion

A lotion was prepared in conventional method according to the same formulation in Example 6, except for using S-equol prepared by the method in Experiment 1 in place of the equol glycoside. When the product was applied to the skin, it exerted effect of whitening the skin and making the skin firm and glossy. Since the product contained equol, it had high preservation stability.

As control, a lotion was prepared by the same method as in Example 6 except for containing no equol glycoside. Thirty volunteers (subjects) were randomly divided in three groups consisting of 10 subjects. Each one of the above three lotions were applied on different area of the medial aspect of the arm of each 10 subjects tree timed a day for 60 days. After two days from the initial application, UV was irradiated on the areas applied with the lotions in a twice dose of the minimal erythema dose (minimum dose of UV which can induce erythema in the UV-irradiated skin), which was previously determined by irradiating various dose of UV to the skin of the subjects. After 24 hours from UV irradiation, the erythema formation was visually observed in the areas applied with the lotions. After 20 days from the initial application, melanin deposition was visually observed. The effect of each lotion was evaluated by the following criteria.

### (Criteria)

### Erythema formation or melanin deposition

Remarkably efficient: Erythema formation or melanin deposition was hardly observed in the area applied with the lotion.

Efficient: Erythema formation ormelanin deposition was distinctly inhibited compared to in the area applied with control.

Not effective: Erythema formation or melanin deposition was observed at the same degree as in the area applied with control.

Oppositely effective: Erythema formation or melanin deposition was enhanced compared to in the area applied with control.

### Experimental result

In regard to erythema formation, when the lotion containing equol glycosides prepared in Example 6 was applied, eight of the 10 subjects answered remarkably efficient or efficient, and when a lotion containing equol prepared in Example 7 was applied, seven of the 10 subjects answered remarkably efficient or efficient. In regard to melanin deposition, when the lotion containing equol glycosides prepared in Example 6 was applied, seven of the 10 subjects answered remarkably efficient efficient, and when a lotion containing equol prepared in Example 7 was applied, seven of the 10 subjects answered remarkably efficient or efficient. These results indicate that since a cosmetic containing an equol compound inhibits melanin deposition as well as skin-inflammation by UV irradiation, it exerts beneficial effect of antiinflammation and whitening. Since the number of the subject who answered remarkably efficient or efficient was about the same when applied the lotion containing either equol or equol glycoside, it is considered that equol and equol glycoside have nearly equol whitening effect. During the test, no skin change such as rough dry skin or rubor was observed in the area applied with any of the lotions.

### Example 8

### Lotion

According to the formulation prescribed below, a lotion was prepared by conventional method.

| (Formulation) | (%) |
|---|---|
| Squalene | 8 |
| Polyoxyethylenesorbitol tetraoleate | 0.3 |
| Sorbitol | 34 |
| Pullulan | 0.001 |
| Ethyl alcohol | 1 |
| 7-O-α-D-Glucopyranosyl-S-equol prepared | 0.5 |
| by the method in Example 3 | |
| L-ascorbic acid 2-glucoside | 2 |
| Haemostatic (calamine) | 0.1 |
| Antiseptic | Adequate |
| Flavor | Adequate |
| Purified water | Remaining |

The total amount was made into 100%.

The product is a beneficial lotion that exerted effect of whitening the skin and making the skin firm and glossy when applied to the skin. Since the product contained 7-O-α-D-glucopyranosyl-S-equol, it had high preservation stability.

### Example 9

### Lotion

According to the formulation in Example 8 except for using R-equol prepared by the method in Experiment 1 in place of equol glycoside, a lotion was prepared by conventional method. The product was a beneficial lotion that exerted effect of whitening the skin and making the skin firm and glossy when applied to the skin. Since the product contained equol, it had high preservation stability.

### Example 10

### Milky lotion

According to the formulation prescribed below, a milky lotion was prepared by conventional method.

| (Formulation) | (%) |
|---|---|
| Polyoxyethylenesorbitan monostearate | 1 |
| (20 E.O.) | |
| Polyoxyethylenesorbitol tetrastearate | 0.5 |
| (60 E.O.) | |
| Glycerol monostearate | 1 |
| Stearic acid | 0.5 |
| Behenyl alcohol | 0.5 |
| Squalene | 8 |
| 4'-O-α-D-Glucopyranosyl-S-equol prepared | 1 |
| by the method in Example 3 | |
| Antiseptic | 0.1 |
| Carboxyvinylpolymer | 0.1 |
| Sodium hydroxide | 0.05 |
| Ethanol | 5 |
| Purified water | Remaining |
| Flavor | Adequate |

The total amount was made into 100%.

The product was a beneficial milky lotion that exerted effect of whitening the skin, diminishing the skin wrinkles, and making the skin firm and glossy when applied to the skin.

### Example 11

### Milky lotion

According to the formulation prescribed below, a milky lotion was prepared by conventional method.

| (Formulation) | (%) |
|---|---|
| Polyoxyethylene(20)polyoxypropylene(2) | 1 |
| cetylether | |
| "KF96-A" (methyl polysiloxane commercialized | 2 |
| by Shin-Etsu Chemical Co., Ltd.) | |
| Liquid paraffin (medium viscosity) | 3 |
| Propyleneglycol | 5 |
| 4'-O-α-D-Glucopyranosyl-R-equol prepared | 1 |
| by the method in Example 2 | |
| Powdery product of indigo plant extract | 0.5 |
| (produced by Hayashibara Biochemical | |
| Laboratories, Inc.) | |
| Glycerol | 2 |
| Ethyl alcohol | 15 |
| Carboxyvinylpolymer | 0.25 |
| Hydorxypropylcellulose | 0.1 |
| Pullulan | 0.05 |
| 2-Aminomethylpropanol | 0.1 |
| Antiseptic | Adequate |
| Purified water | Remaining |

The total amount was made into 100%.

The product was a beneficial milky lotion that exerted effect of whitening the skin, diminishing the skin wrinkles, and making the skin firm and glossy when applied to the skin.

### Example 12

### Milky lotion

According to the formulation in Example 11 except for using the mixture of R-equol and S-equol used in Experiment 1 in place of equol glycoside, a milky lotion was prepared by conventional method. The product was a beneficial milky lotion that exerted effect of whitening the skin, diminishing the skin wrinkles, and making the skin firm and glossy when applied to the skin. Since the product contained equol, it had high preservation stability.

### Example 13

### Cream

According to the formulation prescribed below, a cream was prepared by conventional method.

| (Formulation) | (%) |
|---|---|
| Decaglyceryl monomyristate | 3 |
| Purified lanolin | 0.5 |
| 2-octyldodecanol | 2 |
| Cetyl 2-ethylhexanate | 3 |
| Hexamethyltetracosane | 5 |
| Methyl polysiloxane | 0.3 |
| Behenyl alcohol | 3 |
| Cetanol | 2 |
| Glycerol monostearyl alcohol | 1 |
| Cetyl palmitate | 2 |
| Glycerol monostearate | 2.3 |
| 4'-O-α-D-Glucopyranosyl-R-equol prepared | 1 |
| by the method in Example 2 | |
| 1,3-Butyleneglycol | 5 |
| 1,2-Pentandiol | 3.5 |
| Glycerol | 5 |
| "TORNARE" (glycosyltrehalose/hydrogenated | 2 |
| starch hydrolysate commercialized by | |
| Hayashibara Biochemical Laboratories, Inc.) | |
| Glucosyl rutin | 0.05 |
| 1% Citric acid | 1 |
| Purified water | Remaining |

The total amount was made into 100%.

The product was a beneficial cream that exerted effect of whitening the skin, diminishing the skin wrinkles, and making the skin firm and glossy when applied to the skin.

### Example 14

### Cream

According to the formulation in Example 13 except for using S-equol prepared by the method in Experiment 1 in place of equol glycoside, a cream was prepared by conventional method. The product was a beneficial cream that exerted effect of whitening the skin, diminishing the skin wrinkles, and making the skin firm and glossy when applied to the skin. Since the product contained S-equol, it had high preservation stability.

### Example 15

### Hair tonic

According to the formulation prescribed below, a hair tonic was prepared by conventional method.

| (Formulation) | (%) |
|---|---|
| *"Kankoso"* 301 | 0.005 |
| Sialid extract | 3 |
| Dipotassium glycyrrhizinate | 0.1 |
| Hydrous crystalline α,α-trehalose | 0.03 |
| "α-GLUCOSYLHESPERIDINE" (glycosyl hesperidin | 0.01 |
| commercialized by Hayashibara Biochemical | |
| Laboratories, Inc.) | |
| 7-O-α-D-Glucopyranosyl-R-equol prepared | 0.15 |
| by the method in Example 2 | |
| Seaweed extract | 0.75 |
| Glycerol | 2 |
| Ethanol | 45 |

The total amount was made into 100% with purified water.

The hair tonic exerts inhibitory effect on inflammation or aging of scalp, hair growing and restoring effect, inhibitive effect on dandrull and itch, and effect of keeping the scalp clean since it contains 7-O-α-D-glucopyranosyl-R-equol. When the product is applied to the scalp into which hair root was transplanted, it exerts beneficial effect of enhancing the growth of hair papilla cell and effect of elevating roothold-ratio of hair root on the scalp.

### Example 16

### Sunscreen

According to the formulation prescribed below, a sunscreen was prepared by conventional method.

| (Formulation) | (%) |
|---|---|
| Purified water | 35 |
| 1,3-Butyleneglycol | 5 |
| Octyl squalene-p-methoxycinnamate | 6 |
| Oxybenzene | 3 |
| Hydorphobic titanium dioxide | 3 |
| Glycerol diisostearate | 3 |
| Ascorbic acid 2-glucoside (commercialized by | 2 |
| Hayashibara Biochemical Laboratories, Inc.) | |
| Silk powder supporting gardenia yellow | 3 |
| (commercialized by Hayashibara Biochemical | |
| Laboratories, Inc.) | |
| 4'-O-α-D-Glucopyranosyl-R-equol prepared | 0.15 |
| by the method of Example 3 | |
| Organic-modified montmorillonite | 1.5 |

Since the sunscreen contains equol glycoside, it exerts effect of whitening the skin, diminishing wrinkles of the skin, and making the skin firm and glossy. Since it also contains silk powder supporting gardenia yellow, having effect of antioxidant, radical-trapping, inhibiting elastase activity or lipase activity, the product is usable to inhibit the formation of acne or wrinkle and to keep the skin firm and dullness-free by enhanced effect of equol glycoside.

### Example 17

### Lipstick

According to the formulation prescribed below, a lipstick was prepared by conventional method.

| (Formulation) | (%) |
|---|---|
| Powdery product containing equol glycoside | 1.5 (as equol) |
| prepared by the method in Example 1 | |
| "HAYASHIBARA OUNI SILK" (silk powder | 1 |
| supporting gardenia yellow and safflower red | |
| (commercialized by Hayashibara Biochemical | |
| Laboratories, Inc.) | |
| Titanium dioxide | 3.5 |
| Food Blue No.1 (Brilliant Blue FCF) | 1 |
| Black iron oxide | 0.1 |
| Colcothar | 1.5 |
| Castor oil | 24 |
| Candelilla wax | 8 |
| "TORNARE" (saccharide product containing | 1 |
| saccharide derivatives of α,α-trehalose | |
| commercialized by Hayashibara Biochemical | |
| Laboratories, Inc.) | |
| Solid paraffin | 8 |
| Beewax | 5 |
| Carnauba wax | 5 |
| Lanolin | 11 |
| Cetyl 2-ethylhexanoate | 20 |
| Isopropyl myristate | 10 |
| Antioxidant | Adequate |
| Flavor | Adequate |

Since the lipstick exerts effect of enhancing blood flow in the skin and inhibiting formation of active oxygen and lipid peroxide, as well as of reducing dullness in lip or its neighbor skin, it prolongs the effect of inhibiting the formation of whrinkle and laughter lines in the lip or the neighbor skin and of inhibiting the aging. Since it also contains silk powder supporting gardenia yellow and safflower red, having effect of antioxidant, radical-trapping, inhibiting elastase activity or lipase activity, the product is used to keep the lip without wrinkles, laughter lines and dullness.

### Example 18

### Powdery foundation

According to the formulation prescribed below, a lipstick was prepared by conventional method.

| (Formulation) | (%) |
|---|---|
| Talc | 20 |
| Mica | 33 |
| Kaolin | 7 |
| Nylon powder | 10 |
| Titanium dioxide | 10 |
| Titanated mica | 3 |
| Zinc stearate | 1 |
| Red iron oxide | 1 |
| Yellow iron oxide | 3 |
| Powdery product containing equol glycoside | 1.5 (as equol) |
| prepared by the method in Example 1 | |
| "HAYASHIBARA SHIKON BLACK" (chitosan powder | 1 |
| stained with Shikon commercialized by | |
| Hayashibara Biochemical Laboratories, Inc.) | |
| "HAYASHIBARA OUNI CELLULOSE" (cellulose | 2 |
| supporting gardenia yellow and safflower red | |
| commercialized by Hayashibara Biochemical | |
| Laboratories, Inc.) | |
| Glycosilated hesperidine | 3 |
| Squalene | 6 |
| Lanoline acetate | 1 |
| Octyldodecyl myristate | 2 |
| Flavor | Adequate |
| Antiseptic | Adequate |

Since the powdery foundation contains equol glycoside, it exerts effect of whitening the skin, diminishing wrinkles of the skin, and making the skin firm and glossy. Since it also contains cellulose powder supporting gardenia yellow, having effect of antioxidant, radical-trapping, inhibiting elastase activity or lipase activity, the product is used to inhibit the formation of acne or wrinkle and to keep the skin firm by enhanced effect of equol glycoside.

### Example 19

### Soap

Ninety six and fife tenth parts by weight of neat soap, made from mixture of beef tallow and palm oil in a mass ratio of 4:1 by conventional saponifying and salting method, was homogeneously admixed with two parts by weight of the powdery product containing equol and equol glycoside prepared by the method in Example 4, 1.5 pats by weight of pulverized "HALODEX" (saccharide product containing saccharide derivatives of α,α-trehalose commercialized by Hayashibara Biochemical Laboratories, Inc.), 0.5 part by weight of "AA2G" (L-ascorbic acid 2-glucoside commercialized by Hayashibara Biochemical Laboratories, Inc.), 0.5 part by weight of sucrose, 0.5 part by weight of "αG-RUTIN" (glycosyl rutin commercialized by Hayashibara Biochemical Laboratories, Inc.), one part by weight of maltitol, 0.0001 part by weight of "*Kankoso* 201" and adequate amount of flavor, molded, cooled and solidified to obtain a soap. The product exerts beneficial effect of whitening by equol and its glycoside and of slimming, and is comfortable soap without skin desiccation after use.

### Example 20

### Tooth paste

According to the formulation prescribed below, a tooth paste was prepared by conventional method.

| (Formulation) | (%) |
|---|---|
| β-Glycyrrhetinic acid | 0.05 |
| Cetylpyridinium chloride | 0.05 |
| Calcium hydrogen phosphate | 29 |
| 4'-O-α-D-Glucopyranosyl-S-equol prepared | 1 |
| by the method in Example 3 | |
| 7-O-α-D-Glucopyranosyl-R-equol prepared | 1 |
| by the method in Example 2 | |
| Concentrated glycerol | 20 |
| "TORNARE" (saccharide product containing | 10 |
| saccharide derivatives of α,α-trehalose | |
| commercialized by Hayashibara Biochemical | |
| Laboratories, Inc.) | |
| Powdery product of indigo plant extract | 1 |
| (produced by Hayashibara Biochemical | |
| Laboratories, Inc.) | |
| Anhydrous silicic acid | 5 |
| Titanium oxide | 2 |
| Sodium laurylsulfate | 1.2 |
| Sodium carboxymethylcellulose | 1.2 |
| Polyoxyethylene hydrogenated castor oil | 1 |
| Ethanol | 0.5 |
| Propolis extract | 0.5 |
| Magnesium phosphate | 0.3 |
| Sodium lauroylsarcosine | 0.2 |
| Maltitol | 0.1 |
| *p*-Hydroxybenzoate ester | 0.1 |
| Flavor | Adequate |

The total amount was made into 100% with purified water.

Since the tooth paste contains equol glycoside and indigo plant extract, the product is a tooth paste used to keep or improve the oral health by effect of reducing dullness of the gum, keeping the gum firm and ruby, and anti-inflammatory effect. Since the antimicrobial substances in the indigo plant extract bacteriostatic or bactericidal effect on bacteria causing caries or gum disease, it can be advantageously used for inhibiting caries or inhibiting sideration and progress of gum disease.

### Example 21

### Tooth paste

According to the formulation in Example 18 except for using S-equol prepared by the method in Experiment 1 in place of equol glycoside, a tooth paste was prepared by conventional method. Since the tooth paste contains equol glycoside and indigo plant extract, the product is a tooth paste used to keep or improve the oral health by effect of reducing dullness of the gum, keeping the gum firm and ruby, and anti-inflammatory effect. since the antimicrobial substances in the indigo plant extract bacteriostatic or bactericidal effect on bacteria causing caries or gum disease, it can be advantageously used for inhibiting caries or inhibiting sideration and progress of gum disease.

### Example 22

### Mouthwash

According to the formulation prescribed below, a mouthwash was prepared by conventional method.

| (Formulation) | (%) |
|---|---|
| Ethanol | 15 |
| "HALODEX" (syrup containing saccharide | 82 |
| derivatives of α,α-trehalose commercialized | |
| by Hayashibara Biochemical Laboratories, Inc.) | |
| 4'-O-α-D-Glucopyranosyl-S-equol prepared by | 1 |
| the method in Example 3 | |
| 7-O-α-D-Glucopyranosyl-R-equol prepared by | 0.5 |
| the method in Example 2 | |
| "αG-HESPERIDIN" (glycosyl hesperidin | 1 |
| commercialized by Hayashibara Biochemical | |
| Laboratories, Inc.) | |
| Polyoxyethylene hydrogenated castor oil | 2 |
| Saccharin sodium | 0.02 |
| Sodium benzoate | 0.05 |
| Sodium dihydorgen phosphate | 0.1 |
| Artificial color | Adequate |
| Flavor | Adequate |
| Water | 71.7 |

The total amount was made into 100%.

Since the mouthwash contains equol glycoside, the product is a mouthwash used for palliating dry mouth caused by Sjogren's syndrome and for preventing and treating oral inflammation and dysgeusia

When the product was allowed to be used by 15 stomatitis patients twice at every morning and night for 30 days, stomatitis was palliated or cured in 11 of the 15 patients. Dullness of the gum was decreased in 11 of the 15 patients compared to before using the mouthwash.

### Example 23

### Ointment

According to the formulation prescribed below, an ointment was prepared by conventional method.

| (Formulation) | (%) |
|---|---|
| Sodium acetate | 1 |
| Calcium | 4 |
| Glycerol | 10 |
| Peppermint oil | 0.5 |
| 4'-O-α-D-Glucopyranosyl-S-equol prepared | 0.8 |
| by the method in Example 3 | |
| 7-O-α-D-Glucopyranosyl-R-equol prepared | 0.2 |
| by the method in Example 2 | |
| L-ascorbic acid 2-glucoside | 2 |
| Vaseline | 49 |
| Sumac wax | 10 |
| Lanolin | 10 |
| Sesame oil | 10.5 |

The ointment is an external dermatological composition used for whitening and/or beautifying the skin. The produce, having beneficial skin-permeability and spreadability, can be also used for keeping and improving skin health.

### Example 24

### Ointment

According to the formulation in Example 21 except for using S-equol prepared by the method in Experiment 1 in place of equol glycoside, an ointment was prepared by conventional method. The ointment is an external dermatological composition used for whitening and/or beautifying the skin. The produce, having beneficial skin-permeability and spreadability, can be also used for keeping and improving skin health.

### Example 25

### Jelly ointment

According to the formulation prescribed below, a jelly ointment was prepared by conventional method.

| (Formulation) | (%) |
|---|---|
| Glycerol | 44.49 |
| Ethanol | 30 |
| 7-O-α-D-Glucopyranosyl-S-equol prepared | 1 |
| by the method in Example 3 | |
| 7-O-α-D-Glucopyranosyl-R-equol prepared | 0.5 |
| by the method in Example 2 | |
| Powdery product of indigo plant extract | 0.4 |
| (produced by Hayashibara Biochemical | |
| Laboratories, Inc.) | |
| Bittern solution (hardness 54,000 mg/ml) | 5 |
| Gelling agent (High-Vis-wako 104) | 1.5 |
| Polyoxyethylene(20)sorbitanmonooleate | 1.5 |
| *"Kankoso"* 201 | 0.01 |
| 2,2',2" -nitrilotriethanol | 2.5 |

The jelly ointment is an external dermatological composition used for whitening and/or beautifying the skin. The produce, having beneficial skin-permeability and spreadability, can be also used for keeping and improving skin health.

### Example 26

### Bath agent

According to the formulation prescribed below, a bath agent was prepared by conventional method.

| (Formulation) | (%) |
|---|---|
| Hydrous crystalline α,α-trehalose | 74.4 |
| (cosmetic grade, commercialized by | |
| Hayashibara Biochemical Laboratories, Inc.) | |
| Sodium hydrogen carbonate | 12.5 |
| Powdery product containing equol glycoside | 2.5 |
| prepared by the method in Example 1 | |
| Powdery product containing α,α-trehalose | 5.5 |
| and bittern in a mass ratio of 144:202 | |
| prepared by the method in Example 9 | |
| described in International Patent Publication | |
| WO 2003/0165325 | |
| "α-GLUCOSYLHESPERIDIN" (glycosyl hesperidin | 3 |
| commercialized by Hayashibara Biochemical | |
| Laboratories, Inc.) | |
| *"Kankoso"* 201 | 0.0015 |
| Flavor | Adequate |
| Dye | Adequate |

The total amount was made into 100%.

Since the bath agent containing equol and its glycoside, it exerts beneficial effect of slimming, whitening and beautifying skin.

### Example 27

### Mouth refrigerating film

According to the formulation prescribed below, a mouth refrigerating film was prepared by conventional method.

| (Formulation) | (%) |
|---|---|
| Pullulan (food additive grade, | 22 |
| commercialized by Hayashibara Shoji, Inc.) | |
| Carrageenan | 1 |
| Xanthan gum | 0.15 |
| Locust bean gum | 0.15 |
| Maltitol | 0.8 |
| Deionized water | 69.25 |
| Powdery product containing equol glycoside | 3 |
| prepared by the method in Example 1 | |
| Emulsified mint oil | 2.6 |
| Propolis extract | 0.5 |
| Sucralose | 0.3 |
| Citric acid | 0.25 |

Since the mouth refrigerating film can exert beneficial effect of whitening and/or improving lipid metabolism, reducing dullness of the gum, keeping the gum firm and ruby, and antiinflammation by equol and its glycoside contained therein, it is used for keeping and improving mouth health. The product is also used as breath film.

### Example 28

### Chewing gum

Forty five parts by weight of gum base, 54.7 parts by weight of sucrose, 10 parts by weight of powdery product containing equol glycoside prepared by the method in Example 1, "HAYASHIBARA HESPERIDIN S" (glycosyl hesperidin commercialized by Hayashibara Shoji, Inc.), adequate amount of flavor and dye were mixed and the total amount was made into 100% by conventional method to obtain a chewing gum.

Since the chewing gum can exert beneficial effect of whitening and/or improving lipid metabolism, reducing dullness of the gum, keeping the gum firm and ruby, and antiinflammation by glycosyl hesperidin, equol and its glycoside contained therein, it is used for keeping and improving mouth health. The product is also used as breath film.

### Example 29

### Candy

Ninety five parts by weight of "MABIT POWDER" (powdery sacccharide containing 93.5% maltitol, on a dry solid basis, commercialized by Hayashibara Shoji, Inc.), seven parts by weight of "HALODEX" (syrup containing saccharide derivatives of α,α-trehalose commercialized by Hayashibara Biochemical Laboratories, Inc.) were dissolved in 32 parts by weight of water by heating, and boiled under reduced pressure until the temperature reached about 160°C. Just after boiling, the resultant syrup was admixed with 0.1 part by weight of peppermint oil, 0.1 part by weight of *"nanten"* extract, 0.1 part by weight of *"karin"* extract, 1 part by weight of 4'-O-α-D-glucopyranosyl-S-equol and 7-O-α-D-glucopyranosyl-S-equol prepared by the method in Example 3. The syrup was molded by deposit method by 3 g per drop to give a candy

Since the candy exerts beneficial effect of whitening and/or improving lipid metabolism, antiinflammation, palliating inflammatory symptoms of epidermal cell of respiratory organs such as oral cavity, throat and trachea, palliating inflammation caused by gum disease or its cause, and preserving oral cavity and its nearby cells and tissue, the product can be used as throat lozenge for palliating respiratory diseases such as cold or allergy, palliating pain or discomfort of throat or oral cavity caused by air pollution, exhaust gas or smoke of tobacco, and preventing and palliating gum disease. The product can be also used for whitening and beautifying the skin, preventing and palliating obesity or diabetes.

### Example 30

### Candy

According to the formulation in Example 30 except for using S-equol prepared by the method in Experiment 1 in place of equol glycoside, a candy was prepared by conventional method.

Since the candy exerts beneficial effect of whitening and/or improving lipid metabolism, antiinflammation, palliating inflammatory symptoms of epidermal cell of respiratory organs such as oral cavity, throat and trachea, palliating inflammation caused by gum disease or its cause, and preserving oral cavity and its nearby cells and tissue by equol contained therein, the product can be used as throat lozenge for palliating respiratory diseases such as cold or allergy, palliating pain or discomfort of throat or oral cavity caused by air pollution, exhaust gas or smoke of tobacco, and preventing and palliating gum disease. The product can be also used for whitening and beautifying the skin, preventing and palliating obesity or diabetes.

### Example 31

### Health supplement

Five parts by weight of coenzyme Q₁₀, 26 parts by weight of "HAYASHIBARA ROYAL JELLY EXTRACT X" (royal jell extract commercialized by Hayashibara Shoji, Inc.), five parts by weight of powdery sugar, 50 parts by weight of erythritol, eight parts by weight of ascorbic acid 2-glucoside, one part by weight of vitamin B1, one part by weight of vitamin B2, one part by weight of vitamin B6, three parts by weight of powdery product containing equol and equol glycoside prepared by the method in Example 4, one part by weight of "HAYASHIBARA HESPERIDIN S" (glycosyl hesperidin commercialized by Hayashibara Shoji, Inc.) and one part by weight of flavor were mixed, then a granular health supplement was prepared from the mixture by extruding granulation method and fluid drying method.

Since the health supplement exerts beneficial effect of whitening and/or improving lipid metabolism, antiinflammation, palliating inflammatory symptoms of epidermal cell of respiratory organs such as oral cavity, throat and trachea, palliating inflammation caused by gum disease or its cause, and preserving oral cavity and its nearby cells and tissue by equol and its glycoside contained therein, the product can be used as throat lozenge for palliating respiratory diseases such as cold or allergy, palliating pain or discomfort of throat or oral cavity caused by air pollution, exhaust gas or smoke of tobacco, and preventing and palliating gum disease. The product can be also used for whitening and beautifying the skin, preventing and palliating obesity or diabetes.

### INDUSTRIAL APPLICABILITY

The whitening and/or the lipid metabolism improving agents of the present invention can be used as external dermatological compositions such as cosmetics, quasi-drugs and medicines, or as orally-administered compositions such as foods and beverages, medicines and quasi-drugs. Since equol and/or equol glycoside have estrogenic activity, whitening activity, activity of inducing differentiation of precursor lipocyte, activity of enhancing decomposition of triglyceride in lipocyte and antioxidation activity at once, the whitening and/or lipid metabolism improving compositions of the present invention are used for whitening skin, preventing or palliating menopausal disease and osteoporosis, preventing or treating aging or wrinkle formation in the skin, palliating allergic symptoms, improving lipid metabolism, preventing obesity, preventing or palliating diabetes, preventing and treating cardiac or vascular diseases, and preventing and palliating symptoms caused by acute decrease of estrogen in ovaries. The present invention exerts remarkable effects described above, and is a significantly important invention that greatly contributes to the art.

## Claims

1. A whitening agent comprising equol and/or equol glycoside represented by the following General Formula 1 as an effective ingredient(s). 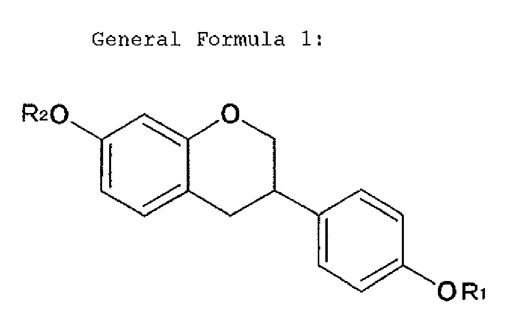 (R₁ and R₂ independently represent a hydrogen atom or a glycosyl group)

2. The whitening agent of claim 1, wherein said equol glycoside is α-glycosyl-equol.

3. The whitening agent of claim 2, wherein the glycosyl group of said α-glycosyl-equol is a glucosyl group and/or a maltosyl group.

4. The whitening agent of claim 3, wherein said α-glycosyl-equol is one or more members selected from the group consisting of 4'-O-α-D-glucopyranosyl-equol, 7-O-α-D-glucopyranosyl-equol and 4',7-O-α-D-glucopyranosyl-equol.

5. The whitening agent of any one of claims 1 to 4, wherein said equol or the equol moiety of said equol glycoside is S- and/or R-stereoisomers.

6. A composition for whitening, comprising the whitening agent of any one of claims 1 to 5.

7. The composition of claim 6, having an indication of exerting a whitening effect.

8. The composition of claim 6 or 7, which contains the whitening agent in an amount of 0.0001 % (w/w) or more in terms of the weight of equol.

9. The composition of any one of claims 6 to 8, which is in the form of a food and beverage, cosmetic, quasi-drug, medicine, feed, bait, pet food, or grocery.

10. A use of the whitening agent of any one of claims 1 to 5, or the compositionofanyone of claims 6 to 9 as a lipidmetabolism-improving agent.

11. A process for producing the equol glycoside represented by General Formula 1, comprising a step of allowing either a glycosyltransferase or glycosyltransferase and glucoamylase to act on a solution comprising equol and α-glucosyl saccharide.

12. The process of claim 11, wherein said equol glycoside is α-glycosyl-equol.

13. The process of claim 13, wherein the glycosyl group of said α-glycosyl-equol is glucosyl group and/or maltosyl group.

14. The process of claim 13, wherein said α-glycosyl-equol is one or more members selected from the group consisting of 4'-O-α-D-glycosyl-equol, 7-O-α-D-glycosyl-equol, and 4',7-O-α-D-glycosyl-equol.
